(19) Europäisches Patentamt
European Patent Office
Office européen des brevets

(11) **EP 1 916 249 A1**

(12) **EUROPEAN PATENT APPLICATION**


(43) Date of publication:
**30.04.2008 Bulletin 2008/18**

(21) Application number: **06122058.8**

(22) Date of filing: **10.10.2006**

(51) Int Cl.:
*C07D 471/04* (2006.01) *C07D 417/14* (2006.01)
*C07D 417/06* (2006.01) *C07D 407/06* (2006.01)
*A61K 31/427* (2006.01) *A61P 31/04* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**



(71) Applicant: **LEK Pharmaceuticals d.d.**
**1526 Ljubljana (SI)**

(72) Inventors:
- **Kotnik, Miha**
  **1000 Ljubljana (SI)**
- **Oblak, Marko**
  **1000 Ljubljana (SI)**
- **Stefanic Anderluh, Petra**
  **1000 Ljubljana (SI)**
- **Przelj, Andrej**
  **1000 Ljubljana (SI)**
- **Humljan, Jan**
  **8330 Metlika (SI)**
- **Plantan, Ivan**
  **1291 Skofljica (SI)**
- **Urleb, Uros**
  **1000 Ljubljana (SI)**
- **Solmajer, Tomaz**
  **1000 Ljubljana (SI)**

(74) Representative: **Wichmann, Hendrik**
**Isenbruck Bösl Hörschler Wichmann Huhn**
**Prinzregentenstrasse 68**
**81675 München (DE)**


(54) **3-(benzo[d][1,3]dioxol-5-ylmethyl)-4-(thio)oxo-2-(thio)oxo-azolidin-5-ylidene derivatives as antibacterial agents**


(57) The present invention concerns novel azolidine compounds of formula

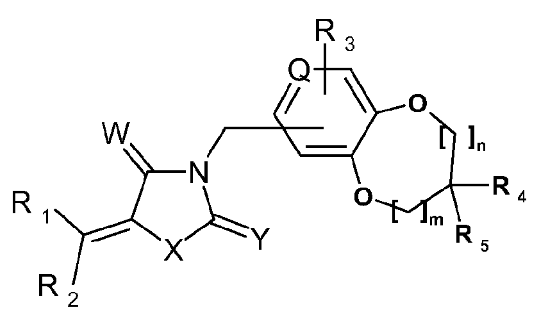

I

wherein X denotes S, NH, $CH_2$ or O, Y and W denotes each independently S or O, Q denotes CH or N, n and m are an integer from 0 to 2, and R1 and R2 denote independently from each other hydrogen, optionally substituted aryl, optionally substituted heterocyclyl, or an optionally substituted ($C_{1-10}$) aliphatic group, R3 denotes hydrogen, lower alkyl, halogen, lower alkoxy, nitro, and R4 and R5 denote independently from each other hydrogen, halogen or alkyl, with the proviso that if one of R1 and R2 is hydrogen then the respective other one of R1 and R2 does not denote substituted or unsubstituted 2-furyland their salts, that are useful as pharmaceutical agents, e.g. as antibacterial agents, and to pharmaceutical compositions comprising such compounds.


EP 1 916 249 A1

## Description

The present invention concerns novel azolidine compounds that are useful as pharmaceutical agents, e.g. as antibacterial agents, and to pharmaceutical compositions comprising such compounds.

The widespread emergence of pathogenic bacterial strains with resistance to antibiotics is becoming a serious threat for public health. As a result of resistance, some bacterial infections are either difficult to treat with existing antibiotics or even untreatable. Recent development of multi drug resistance in certain strains of bacteria, such as methicillin resistant *Staphylococcus aureus* (MRSA), methicillin resistant coagulase negative *Staphylococci* (MRCoNS), penicillin resistant *Streptococcus pneumoniae, Mycobacterium tuberculosis, Pseudomonas aeruginosa,* and multiply resistant *Enterococcus faecium* has risen the seriousness of this problem on a higher level. The appearance of vancomycin resistant *Enterococcus* (VRE) was particularly alarming because vancomycin was formerly the only effective antibiotic for treating this infection. Another resistant hospital-acquired pathogen is carbapenem-resistant *Acinetobacter baumannii.* (A. El Zoeiby, et al., Molecular Microbiology, 2003, 47, 1-12; F. M. Walsh, et al., Current Opinion in Microbiology, 2004, 7, 439-444.)

This situation has generated an urgent need to develop novel broad spectrum antibacterial agents preferably directed towards previously unexplored targets.

Since the essential bacterial cell-wall polymer peptidoglycan is exclusively found in prokaryotic cells the enzymes involved in the peptidoglycan sythesis, such as the Murein ligases MurD, MurC, MurE and MurF are excellent selective targets for an effective antibiotic against prokaryotic organisms.

The present invention relates to novel azolidine compounds being suitable as pharmaceuticals, e.g. as antibacterial agents.

Particularly, the present invention relates to a compound of formula

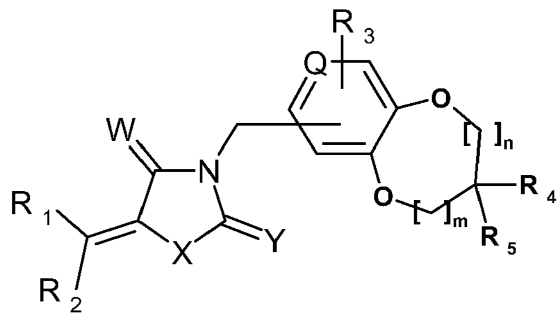

I

wherein

X denotes S, NH, $CH_2$ or O, preferably S or O
Y denotes S or O, preferably S
W denotes S or O, preferably O
Q denotes CH or N, preferably CH
n is an integer from 0 to 2,
m is an integer from 0 to 2, whereby the sum of n+m is not greater than 2, e.g. n is 1 and m is 1, or preferably n is 0 and m is 0,
R1 and R2 denote independently from each other hydrogen, optionally substituted aryl, optionally substituted heterocyclyl, or an optionally substituted ($C_{1-10}$) aliphatic group,
R3 denotes hydrogen, lower alkyl, halogen, lower alkoxy, nitro, and
R4 and R5 denote independently from each other hydrogen, halogen or alkyl, with the proviso that if one of R1 and R2 is hydrogen then the respective other one of
R1 and R2 does not denote substituted or unsubstituted 2-furyl,

and pharmaceutically acceptable salts of a compound of formula I.

As used herein the following definitions shall apply unless otherwise specifically indicated. The term aliphatic means straight chained, branched or cyclic hydrocarbons which are completely saturated or which contain one or more units of unsaturation. For example, suitable aliphatic groups include substituted or unsubstituted linear, branched or cyclic alkyl, alkenyl, alkinyl groups and hybrids thereof such as (cycloalkyl)alkyl, (cycloalkenyl)alkyl or (cycloalkyl)alkenyl. Alkyl includes ($C_{1-20}$)alkyl, particularly ($C_{1-8}$)alkyl, such as lower alkyl. Lower alkyl includes ($C_{1-4}$)alkyl, such as methyl

or ethyl. Alkenyl includes ($C_{2-20}$)alkenyl, particularly ($C_{2-8}$)alkenyl. Alkinyl includes ($C_{2-20}$)alkinyl, particularly ($C_{2-8}$)alkinyl. Cycloalkyl includes ($C_{3-18}$)cycloalkyl, particularly ($C_{3-10}$)cycloalkyl, such as ($C_{3-8}$)cycloalkyl, for example cyclohexyl, cyclopentyl or cyclopropyl.

Alkoxy includes ($C_{1-20}$)alkoxy such as ($C_{1-8}$)alkoxy, e.g. lower alkoxy. Lower alkoxy includes ($C_{1-4}$)alkoxy, in particular methoxy and ethoxy. Halogen includes fluoro, chloro, iodo and bromo such as fluoro and chloro. Alkyl, alkenyl, alkinyl and cycloalkyl include unsubstituted alkyl, alkenyl, alkinyl and cycloalkyl; and substituted alkyl, alkenyl and cycloalkyl, for example, substituted by halogen, hydroxy, alkoxy, amino, nitro, alkylamino, or dialkylamino. Halogenalkyl includes alkyl wherein at least one hydrogen atom and at maximum all halogen atoms are replaced by halogen, e.g. trihalomethyl such as triflouromethyl.

The term heteroatom includes N, O and S.

Aryl includes ($C_{6-18}$)aryl, such as ($C_{6-10}$)aryl. In particular aryl includes phenyl and naphtyl, e.g. phenyl. Aryl may be unsubstituted aryl or substituted aryl, for example by alkoxy, aryloxy, alkylthio, hydroxy, alkyl, amino, alkylamino, dialkylamino, alkylsulfonamido, carboxy, halogen, haloalkyl, cycloalkyl, nitro, cyano, or heterocyclyl. Alkylamino and dialkylamino include alkylamino and dialkylamino wherein the alkyl parts are as defined above.

If not expressly otherwise indicated herein heterocyclyl includes aromatic or alicyclic heterocyclyl being monocyclic and having 3 to 7 members, preferably 5 to 6 members, containing at least one carbon atom and 1 to 4 heteroatoms beside the carbon atoms, or being bicyclic and having 8 to 10 members containing at least 3 carbon atoms and 1 to 7 heteroatoms. Heterocyclyl includes further unsubstituted hetercyclyl and substituted heterocyclyl, for example by oxo, alkoxy, aryloxy, alkylthio, hydroxy, thioxo, alkyl, amino, alkylamino, dialkylamino, alkylsulfonamido, carboxy, halogen, haloalkyl, cycloalkyl, nitro, cyano, isocyano or heterocyclyl. Examples of aromatic heterocyclyl include furanyl; thienyl; pyrrolyl; 2H-pyrrolyl; oxazolyl; thiazolyl; imidazolyl; pyrazolyl; isoxazolyl; isothiazolyl; 1,2,3-oxadiazolyl; 1,2,4-oxadiazolyl; 1,2,4-thiadiazolyl; 1,3,4-oxadiazolyl; 1,3,4-thiadiazolyl; 2H-pyranyl; 4H-pyranyl; 1,4-dioxan; 1,4-dithiane; pyridazinyl; pyrimidinyl, pyrazinyl, 1,2,3-triazinyl, pyridinyl, tetrazolyl. Examples of non-aromatic heterocyclyl include tetrahydrofuranyl, tetrahydrothienyl, morpholinyl, thiomorpholinyl, 2H-pyrrolinyl, 3H-pyrrolinyl, pyrrolidinyl, piperazinyl, piperidinyl, 2-pyrazolinyl, pyrazolidinyl, 3,6-dihydro-2H-pyranyl, 1,2,3,6-tetrahydro-pyridyl, thiazolidinyl, imidazolidinyl, 2-imidazolinyl, diazolone, N-substituted diazolone, phthalimidinyl, benzoxane, benzotriazolyl, benzopyrrolidinyl, benzopiperidinyl, 1,3-dihydroisobenzofuranyl, 1,3-dihydroisobenzothienyl, tetrahydroisoquinolinyl, decahydroisoquinolinyl or benzothianyl.

In a preferred embodiment one of the residues of the group consisting of R1 and R2 denotes hydrogen and the other one is defined as above, e.g. R1 denotes hydrogen and R2 is as defined above, or R2 denotes hydrogen and R1 is as defined above. Preferably R1 or R2 denote aryl or heterocyclyl. Aryl in this connection may be unsubstituted aryl such as phenyl, or substituted aryl, substituted one or several fold, e.g. 1 to 4 fold by hydroxy, alkoxy, alkylthio, aryloxy, alkyl, amino, alkylamino, dialkylamino, nitro, cyano, halogen, carboxy, alkylsulfonamido, heterocyclyl. If R1 or R2 preferably denote heterocyclyl then heterocyclyl may e.g. be pyridinyl, pyrimidinyl, oxazolyl, 1H-indol-3-yl, 1H-benzoimidazol-2-yl, imidazolyl, 1,2,4-triazolyl, benzo[d][1,3]dioxol-5-yl, 1,2,3-thiadiazolyl, thiazolyl, 4H-benzo[d][1.3]dioxinyl or pyrido[1.2a]pyrimidinyl, which may be unsubstituted or substituted one or several fold, e.g. 1 to 4 fold, by lower alkyl, oxo, cyano, nitro, halogen, morpholino, piperazino, hydroxy, alkoxy, aryloxy, amino, alkylamino, dialkylamino, (hydroxyalkyl)amino, (hydroxyalkoxyalkyl)amino or (heterocyclylalkyl)amino.

In a particularly preferred embodiment R1 is hydrogen and R2 is a group of formula

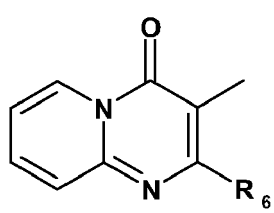

IIa

wherein R6 denotes amino, alkylamino, dialkylamino, (hydroxyalkyl)amino, di(hydroxyalkyl)amino, (heterocyclylalkyl) amino, (hydroxyalkoxyalkyl)amino or di(hydroxyalkoxyalkyl)amino. R6 may preferably be methylamino, ((tetrahydrofuran-2-yl)-methyl)amino, (hydroxyl(lower alkyl))amino or (2-hydroxyethoxy)ethyl)amino.

In another preferred embodiment one residue of the group consisting of R1 and R2 is hydrogen and the other one is aryl or a monocyclic or bicyclic aromatic heterocyclyl with the proviso that R1 or R2 is not 2-furanyl. E.g. aryl or a monocyclic or bicyclic aromatic heterocyclyl for R1 or R2 includes phenyl, pyridinyl, piperonyl, pyrimidinyl, triazinyl, pyrazolyl, imidazolyl, benzimidazolyl, thiazolyl, isothiazolyl, thiadiazolyl, benzodioxinyl, oxazolyl, isoxazolyl or oxadiazolyl, such as phenyl, thiadiazolyl, piperonyl, triazolyl, imidazolyl, benzodioxinyl, oxazolyl or pyridinyl. The aryl or monocyclic or bicyclic aromatic heterocyclyl may be unsubstituted or substituted, for instance 1 to 4 fold, e.g. 1 to 2 fold. Suitable substituents include e.g. halogen, halogenalkyl, alkyl, hydroxy, alkoxy, amino, alkylamino, dialkylamino, nitro,

cyano, alkylsulfonamido, carboxy or heterocyclyl.

R3 in a compound of formula I may preferably denote hydrogen, lower alkyl, halogenalkyl or halogen, e.g. hydrogen, trifluoromethyl, fluoro or chloro.

R4 and R5 in a compound of formula I may preferably denote independently from each other hydrogen, alkyl or halogen, e.g. R4 and R5 denote both hydrogen, lower alkyl or halogen. Most preferably R4 and R5 both denote hydrogen.

A particularly preferred embodiment of the present invention is directed to a compound of formula

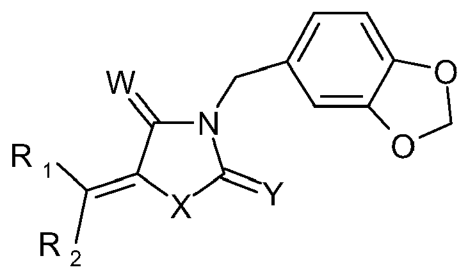

**Ia**

wherein R1, R2, W, X and Y are as defined above.

It will be apparent to one skilled in the art that certain compounds of this invention may exist in tautomeric forms. A compound according to formula I wherein X denotes NH and Y denotes O or S may exist in a lactam and a lactim form respectively in a thiolactam and thiolactim form. All such forms of the compounds according to formula I are understood to be within the scope of the present invention. Unless otherwise stated, structures described herein are also meant to include all stereochemical forms of the structure, e.g. the R/S or E/Z configurations.

Salts of the compounds of formula I, e.g. of formula Ia, include physiologically acceptable acid addition salts for example hydrochlorides, hydrobromides, sulphates, methane sulphonates, p-toluenesulphonates, phosphates, acetates, citrates, succinates, lactates, tartrates, fumarates and maleates. If a compound of formula I includes an acidic group, salts may also be formed with metal ions, for example alkali or earthalkaline salts such as sodium, potassium, magnesium, and calcium salts.

Compounds of formula I may be prepared from an intermediate compound of formula

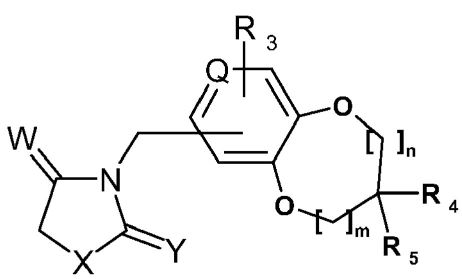

**V**

wherein W, X, Y, Q, R3, n, m, R4 and R5 are as defined above in formula I, by reacting a compound of formula V with an aldehyde or ketone of formula

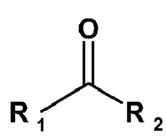

**VI**

wherein R1 and R2 are defined as in formula I, to obtain a compound of formula I.

The reaction of a compound of formula V with a compound of formula VI may be carried out in analogy to known methods, e.g. by Knoevenagel condensation, such as heating under reflux or microwave irradiating of a mixture of a compound of formula V with a compound of formula VI in the presence of trialkylamine, e.g. triethylamine, and an alkanol, e.g. ethanol. An alternative way for reacting a compound of formula V with a compound of formula VI may be by heating or

microwave irradiation in the presence of an acetate salt and acetic acid analogously as for instance described in Orchard M.G. et al, Bioorg. Med. Chem. Lett. 2004, 14, 3975-3978.
A compound of formula V may be prepared in analogy to known methods, for instance a compound of formula V wherein X denotes NH and W, Y, Q, R3, R4 and R5 are as defined in formula I above may be prepared by cyclization of a compound of formula

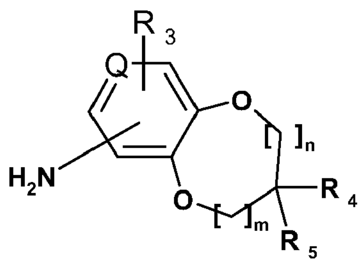

VII

wherein Q, n, m, R4 and R5 have the same meaning as defined above in formula I with a compound of formula

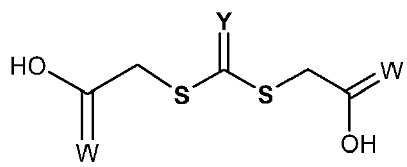

VIII

wherein Y and W are as defined above in formula I, to obtain a compound of formula V. This cyclization step may be carried out under conditions in analogy to, e.g. according to known processes, such as described in Woolhouse A.D. et al, J. Mater. Chem. 2001, 11, 2271-2281.

A compound of formula V wherein W, X, Z, Q, R3, n, m, R4 and R5 are as defined above in formula I may also be prepared by reacting a compound of formula

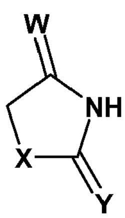

IX

wherein W, X and Y are as defined above in formula I, with a compound of formula

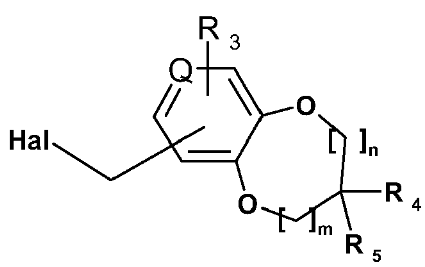

X

wherein R3, Q, n, m, R4 and R5 are as defined in formula I above and Hal denotes halogen, in order to obtain a compound of formula V. Such a reaction may be carried out under conditions in analogy, e.g. according to conventional preparation methods, e.g. by suspending a compound of formula IX in a suitable solvent, for instance an aprotic solvent such as DMSO, DMF or acetonitrile, and then adding a compound of formula X followed by stirring the suspension in the presence of a base such as DBU, alkali carbonates, alkali hydrides or alkali fluoride at room temperature. In order to isolate a

compound of formula V from the reaction mixture the solvent may be removed by conventional methods, e.g. evaporation under reduced pressure, and a compound of formula V may optionally be purified by conventional methods, e.g. by column chromatography on silicagel.

Alternatively, a compound of formula V wherein X denotes S, O or NH and W, Y, Q, R3, n, m, R4 and R5 are as defined above in formula I may be prepared by reacting a compound of formula

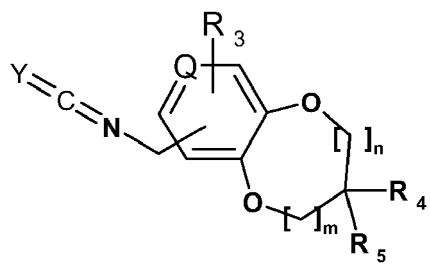

III

wherein Y, Q, R3, R4, R5, n and m have the same meaning as defined above in formula I, with a compound of formula

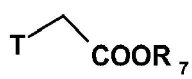

IV

wherein R7 denotes alkyl and T denotes OH, SH or $NH_2$, e.g. under conventional conditions as described in Sim M.M. et al, Bioorg. Med. Chem. Lett. 2001, 11, 91-94.

A compound of formula I wherein X denotes $CH_2$ and W, Y, Q, R3, n, m, R4 and R5 are as defined above in formula I may also be prepared by reacting a compound of formula

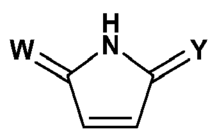

XI

wherein W and Y have the same meaning as in formula I with a compound of formula

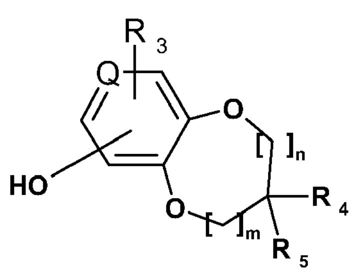

XII

to obtain a compound of formula

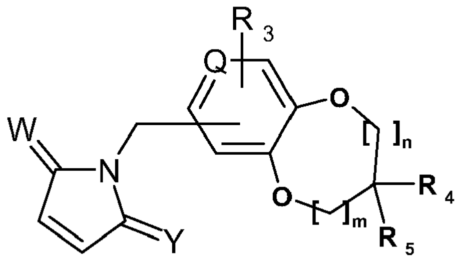

XIII

which may then be further reacted with a compound of formula

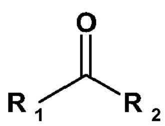

VI

wherein R1 and R2 have the same meaning as defined above in formula I to obtain a compound of formula I wherein X denotes CH. The reaction of a compound of formula XI with a compound of formula XII may be carried out analogously to known methods, e.g. under conditions as known for Mitsunobu reactions, by adding di(lower alkyl)azodicarboxylate to a solution of a compound of formula XI and a compound of formula XII in a suitable solvent, e.g. THF, in the presence of triphenylphosphine and stirring that reaction mixture e.g. at room temperature, followed by removing the solvent, e.g. under reduced pressure by evaporation and finally purifying the obtained residue, e.g. by chromatography to isolate a compound of formula XIII.

The reaction of a compound of formula XIII with a compound of formula VI may also be carried out in analogy, e.g. according to known processes, for example by first heating a solution of a compound of formula XIII in the presence of triphenylphosphine in a suitable solvent, e.g. acetic acid, under reflux, removing the solvent and then suspending the obtained residue into a suitable solvent, e.g. toluene, in order to add a compound of formula VI and facilitating the reaction by increasing the temperature to e.g. 90-150˚C. The obtained compound of formula I wherein X is CH may further optionally be purified by conventional methods, e.g. filtering, washing and drying.

A compound of formula VII wherein R3, n, m, R4 and R5 are as defined above in formula I may be prepared by cyclisation of a corresponding 3,4-dihydroxyaldehyde and a suitable ketone, dialkyl ketal or alkyl enolether under conditions known in the field (e.g. as decribed in Jae-Kon et. all. Bulletin of the Korean Chemical Society (2002), 23(5), 661-662; Yasuyuki Kita et all. JOC 1998, 63, 6625-6633; Reddy TJ et al. JOC 2002, 67, 4635) followed by formation of an oxime by adding hydroxylamine in the presence of a base such as NaOH, triethylamine etc. (J. S. Buck, W. S. Ide. Organic Syntheses, Coll. Vol. 2, p.622 (1943); Vol. 15, p.85 (1935).) and reduction by catalytic hydrogenation or hydride complex reducing agents (DIBAL, $LiAlH_4$, $NaBH_4$, etc.) (M. Schlosser, J. Gorecka, E. Castagnetti. Eur. J. Org. Chem. 2003, 452-462).

Thus, in a further aspect the present invention provides a process for the preparation of a compound of formula I, e.g. a compound of formula Ia, as defined above comprising the steps of

a) reacting a compound of formula V as defined above with an aldehyde or ketone of formula IV as defined above to obtain a compound of formula I, and optionally
c) isolating a compound of formula I

In another aspect the present invention provides a process for the preparation of a compound of formula I wherein X denotes S, O or NH comprising the steps of

t) reacting a compound of formula III as defined above with a compound of formula VI as defined above to obtain a compound of formula V as defined above wherein X denotes S, O or NH,
b) reacting a compound of formula V as defined above with an aldehyde or ketone of formula IV as defined above to obtain a compound of formula I, and optionally
c) isolating a compound of formula I.

In another aspect the present invention relates to a process for the preparation of a compound of formula I as

defined above wherein X denotes $CH_2$ comprising the steps of

i) reacting a compound of formula XI as defined above with a compound of formula XII as defined above to obtain a compound of formula XIII as defined above,

ii) reacting a compound of formula XIII as defined in step i) with a compound of formula VI

wherein R1 and R2 have the same meaning as defined above in formula I to obtain a compound of formula I wherein X denotes CH, and optionally iii) isolating a compound of formula I wherein X denotes CH.

Step c), respectively step iii), the isolation of a compound of formula I, in the described processes may be carried out in analogy to, e.g. according to conventional methods for the isolation of compounds from reaction solutions, such as chromatography, filtration and/or drying.

The compounds according to formula I, e.g. those according to formula Ia, exhibit pharmacological activity and are therefore useful as pharmaceuticals. In particular, compounds of formula I show inhibitory activity against Mur ligases such as MurD and/or MurE. For instance, (Z)-3-(benzo[d][1,3]dioxol-5-yl-methyl)-5-(2-hydroxybenzylidene)-2-thioxothiazolidin-4-one (compound of Ex. 8) shows an $IC_{50}$ concentrations (inhibitory concentration required for 50% inhibition of enzyme activity) against MurE from *S.aureus* of 1.5μM and (Z)-3-(benzo[d][1,3]dioxol-5-yl-methyl)-5-((4-oxo-2-((tetrahydrofuran-2-yl)methylamino)-4H-pyrido[1,2-a]pyrimidin-3-yl)methylene)-2-thioxothiazolidin-4-one (compound of Ex. 3) at 100μM concentration leads to a residual activity of MurD from E.coli of 7% when tested in an MurD enzyme assay. Mur ligases (MurC, MurD, MurE, MurF) catalyse the stepwise addition of L-alanine, D-glutamic acid, a diamino acid (usually diaminopimelic acid or L-lysine), and a dipeptide D-alanine-X (wherein X is usually D-alanine and less frequently X is D-lactate or D-serine) onto the D-lactoyl group of cytoplasmic peptidoglycan precursor UDP-MurNAc. (J. A. Bertrand, et al., Journal of Molecular Biology, 1999, 289, 579-590)

Among Mur family enzymes, UDP-*N*-acetylmuramoyl-L-alanin:D-glutamate ligase (MurD) appears to represent a very good target for an antibiotic agent due to the presence of L-Ala-D-Glu link in every eubacterial cell. In addition, D-amino acid residues cannot be found in eukaryotic cells. Therefore, MurD inhibitors such as the compounds of formula I according to the present invention may possess very good properties with respect to selective toxicity.

MurE synthetase catalyses the addition of either meso-diaminopimelic acid or L-lysine to the substrate UDP-*N*-acetylmuramoyl-L-alanin:D-glutamate (depending on the organism). Rarely, an ornithine, diaminobutyric acid, homoserine, lanthionine, or 3-hydroxy-2,6-diaminopimelic acid is located at position 3 in the peptide subunit (J. Van Heijenoort, Natural Product Reports, 2001, 18, 503-519). In general, the name MurE stands for UDP-*N*-acetylmuramoyl-L-alanin-D-glutamate:meso-diaminopimeate ligase or UDP-*N*-acetylmuramoyl-L-alanin-D-glutamate:L-lysine ligase, depending which residue the enzyme adds to the growing peptide chain.

In another aspect the present invention provides thus a compound of formula I, e.g. of formula Ia, as a pharmaceutical, preferably as an antimicrobial agent, such as an antibiotic.

In a further aspect the present invention provides a compound of formula I, e.g. of formula Ia for use in the preparation of a medicament for the treatment of microbial diseases,

In a further aspect the present invention is directed to the use of a compound of formula I in the preparation of a medicament for the treatment of microbial diseases.

The term microbial diseases includes diseases caused by bacteriae having Mur ligases, e.g. having MurD and/or MurE ligases such as those bacteria which are sensitive to MurD inhibitors and/or MurE inhibitors for example bacteria of strains *E.coli, E. faecalis S. aureus* including MRSA strains.

In a further aspect the present invention provides a method of treatment of microbial, e.g. bacterial, diseases which comprises administering to a subject in need of such treatment an effective amount of a compound of formula I.

The appropriate dosage and treatment regimens for any particular patient will depend upon a variety of factors, including the activity of the specific compound employed, the age, body weight, general health status, sex, diet, time of administration, route of administration, rate of excretion, drug combination, the severity and course of the disease, and the patient's disposition to the disease and the judgment of the treating physician. However, in general, for satisfactory results in larger mammals, for example humans, an indicated daily dosage is in the range from about 0.05 to 5 g, for example 0.1 to about 2.5 g, of a compound of formula I, conveniently administered, for example, in divided doses up to four times a day. Safe and effective dosages for different classes of patients and for different disease states will be determined by clinical trial as is required in the art.

In another aspect the present invention provides a pharmaceutical composition comprising a compound of formula I, e.g. of formula Ia, or a salt thereof. Beside a compound of formula I a pharmaceutical composition according to the present invention may contain one or more pharmaceutical acceptable excipient(s), such as carriers or diluents and optionally additional therapeutically effective compounds. A pharmaceutical composition according to the present invention may be useful for treating bacterial infections.

The term pharmaceutically acceptable excipient refers to conventional excipients as known in the pharmaceutical field, e.g. a non-toxic carrier that may be administered to a patient, together with a compound of formula I, and which does not destroy the pharmacological activity thereof.
The pharmaceutical compositions of this invention may be administered orally, parenterally, by inhalation spray, topically, via ophthalmic solution or ointment, rectally, nasally, buccally, vaginally or via implanted reservoir. The term parenteral as used herein includes subcutaneous, intracutaneous, intravenous, intramuscular, intra-articular, intrasynovial, intrasternal, intrathecal, intralesional and intracranial injection or infusion techniques.

Orally administrable compositions may be for instance in the form of tablets, capsules, powders or granules. Parenterally administrable pharmaceutical compositions may be for instance a suspension or solution of a compound of formula I in a sterile liquid.
The pharmaceutical compositions according to the present invention may be prepared in analogy to, e.g. according to known, conventional methods and techniques in the pharmaceutical field, e.g. analogously to the application of known antibiotics such as Fluorochinolones, e.g. Ciprofloxacin or Enrofloxacin.

Compounds of this invention may be employed in a conventional manner for controlling bacterial infections and for treating diseases or reducing the advancement or severity of effects, which are mediated by bacteria in mammals, e.g. humans or animals. Such methods of treatment, their dosage levels and requirements may be selected by those of ordinary skill in the art from available methods and techniques.

While we have described a number of examples of this invention, it is apparent that our basic constructions may be altered to provide other embodiments which utilize the compounds, processes and compositions of this invention

## Examples

### General procedure A for synthesis of 3-(benzo[d][1,3]dioxol-5-ylmethyl)rhodanines

#### Step 1._Preparation of 3-(benzo[d][1,3]dioxol-5-ylmethyl)-2-thioxothiazolidin-4-one

A suspension of piperonylamine (16.5 mmol, 2.50 g) in 1M NaOH is refluxed for 10 min and cooled to room temperature. Subsequently bis(carboxymethyl) trithiocarbonate (3.74 g, 16.5 mmol) is added and the reaction mixture refluxed overnight to give the precipitate, which is filtered off and ished with water (3x 20 mL). The remaining residue is suspended in a mixture of methanol and isopropanol (1:1, 25 mL) and refluxed for 10 minutes. Cooled reaction mixture is filtered and the solid residue dried at lower temperature and recrystallyzed from methanol to give 3.12 g of 3-(benzo [d][1,3]dioxol-5-ylmethyl)-2-thioxothiazolidin-4-one.

#### Step 2. Coupling of aldehydes or ketones to 3-(benzo[d][1,3]dioxol-5-ylmethyl)-2-thioxothiazolidin-4-one

#### Alternative a) Coupling procedure using triethylamine and ethanol

A solution of 3-(benzo[d][1,3]dioxol-5-ylmethyl)-2-thioxothiazolidin-4-one (100 mg, 0.374 mmol), corresponding aldehyde $R_1CHO$ or ketone $R_1(CO)R_2$ (0.374 mmol) and triethylamine (0.104 mL, 0.748 mmol) in ethanol (3 mL) is heated in the microwave reactor for 2h. The reaction mixture is cooled to room temperature and the obtained precipitate filtered off and washed with EtOH (2x5 mL) and MeOH (2x5mL) and dried at elevated temperature.

#### Alternative b) Coupling procedure using sodium acetate and acetic acid

A solution of 80 mg (=0.299 mmol) 3-(benzo[d][1,3]dioxol-5-ylmethyl)-2-thioxothiazolidin-4-one, corresponding aldehyde R1HCO (0.299 mmol) and sodium acetate (127.4 mg, 1.50 mmol) in acetic acid (3 mL) is heated in the microwave reactor for 2h at around 130°C. The reaction mixture is cooled to room temperature and the obtained precipitate filtered off and washed with EtOH (2x5 mL) and MeOH (2x5mL) and dried at elevated temperature.

### General Procedure B for synthesis of 3-((2,2-dimethylbenzo[d][1,3]dioxol-5-yl)methyl)rhodanines

#### Step 1. Synthesis of 2,2-dimethylbenzo[d][1,3]dioxole-5-carbaldehyde

Phosphorus pentoxide (226 mmol, 32.1 g) is added into a solution of 3,4-dihydroxybenzaldehyde (113 mmol, 15.6 g) and acetone (16.6 mL, 226 mmol) in toluene (230 mL) and stirred at room temperature for 2 hours. Subsequently additional acetone (25 mL) and phosphorus pentoxide (106 mmol, 15.0 g) are added. The reaction mixture is stirred for 2.5 h at room temperature and then poured into a solution of potassium carbonate (140 g) in $H_2O$ (1 L). Ethylacetate is added and the fractions separated. The organic fraction is washed with saturated solution of NaCl, dried over $MgSO_4$ and the solvent removed under reduced pressure. The obtained oily residue is distilled under reduced pressure to yield

2.2 g (11 %) of 2,2-dimethylbenzo[d][1,3]dioxole-5-carbaldehyde.

**Step 2. Synthesis of 2,2-dimethylbenzo[d][1,3]dioxole-5-carbaldehyde oxime**

A solution of sodium hydroxide (18.5 mmol, 0.741 g) in $H_2O$ is added into a solution of 2,2-dimethylbenzo[d][1,3]dioxole-5-carbaldehyde (12.4 mmol, 2.20 g) and hydroxylamine hydrochloride (14.8 mmol, 1.03 g) in ethanol (50 mL) and the reaction mixture stirred overnight. Subsequently diethylether (50 mL) is added and washed with saturated solution of NaCl (2 x 30 mL), dried with $MgSO_4$ and the solvent removed under reduced pressure. The product is washed with hexane to yield 1.87 g (78%) of 2,2-dimethylbenzo[d][1,3]dioxole-5-carbaldehyde oxime.

**Step 3. Synthesis of (2,2-dimethylbenzo[d][1,3]dioxol-5-yl)methanamine**

2,2-Dimethylbenzo[d][1,3]dioxole-5-carbaldehyde oxime (8.85 mmol, 1.71 g) is added into a suspension of $LiAlH_4$ (17.7 mmol, 0.672 g) in dry diethylether (25 mL) and refluxed for 4 hours. After the reaction is complete ethylacetate (60 mL) and water (10 mL) were slowly added into the reaction mixture. The fractions were separated and the organic fraction is additionally washed with saturated solution of NaCl (30 mL) and dried with $MgSO_4$. The solvent is removed under reduced pressure and the oily residue purified with column chromatography (silicagel; ethylacetate/methanol/triethylamine = 1/1/0.01) to yield 0.90 g (57%) of (2,2-dimethylbenzo[d][1,3]dioxol-5-yl)methanamine.

**Step 4. Synthesis of 3-((2,2-dimethylbenzo[d][1,3]dioxol-5-yl)methyl)-2-thioxo-thiazolidin-4-one**

Synthesis of 3-((2,2-dimethylbenzo[d][1,3]dioxol-5-yl)methyl)-2-thioxothiazolidin-4-one is performed in a similar manner as described in example 62.

**Step 5. Coupling of aldehydes to 3-((2,2-dimethylbenzo[d][1,3]dioxol-5-yl)methyl)-2-thioxothiazolidin-4-one.**

Coupling with aldehydes of formula $R_1CHO$ to 3-((2,2-dimethylbenzo[d][1,3]dioxol-5-yl)methyl)-2-thioxothiazolidin-4-one is performed in a similar manner as described in General Procedure A.

**General Procedure C for synthesis of 5-substituted 3-((6-chlorobenzo[d][1,3]dioxol-5-yl)methyl)-2-thioxothiazolidin-4-one**

**Step 1. Synthesis of 3-((6-chlorobenzo[d][1,3]dioxol-5-yl)methyl)-2-thioxothiazolidin-4-one**

A suspension of thiazolidine-2,4-dione (5.0 mmol, 0.586 g), 5-chloro-6-(chloromethyl)benzo[d][1,3]dioxole (5.0 mmol, 1.03 g) and potassium fluoride (5.0 mmol, 0.291 g) in DMF (10 mL) is stirred at room temperature overnight. The solvent is removed under reduced pressure and the residue purified by column chromatography (silicagel; ethylacetate/hexane = ¼) to give 0.649 g (45%) of 3-((6-chlorobenzo[d][1,3]dioxol-5-yl)methyl)-2-thioxothiazolidin-4-one.

**Step 2. Coupling of aldehydes to 3-((6-chlorobenzo[d][1,3]dioxol-5-yl)methyl)-2-thioxothiazolidin-4-one**

Coupling of aldehydes to 3-((6-chlorobenzo[d][1,3]dioxol-5-yl)methyl)-2-thioxothiazolidin-4-one is performed in a similar manner as described in General Procedure A.

**Table 1:** The compounds of Examples 1 to 50 and 55 to 61 are prepared according to the General Procedure A and compounds of Examples 51 to 52 according to General Procedure B as set out above from reactions with corresponding aldehydes of formula $R_1HCO$. Compound of Example 54 is prepared according to process of Example 62; compound of Example 55 is prepared according to General Procedure C as described above

| Ex. No. | $R_1$ | Structure | Name |
|---|---|---|---|
| 1 | 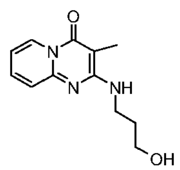 | 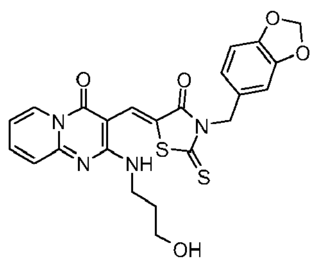 | (Z)-3-(benzo[d][1,3] dioxol-5-yl-methyl)-5-((2-(3-hydroxypropylamino)-4-oxo-4*H*-pyrido[1,2-a] pyrimidin-3-yl) methylene)-2-thioxothiazolidin-4-one |
| 2 | 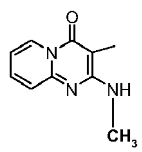 | 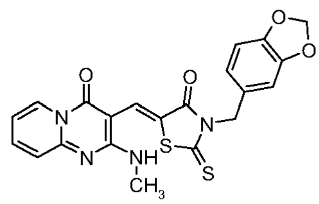 | (Z)-3-(benzo[d][1,3] dioxol-5-yl-methyl)-5-((2-(methylamino)-4-oxo-4*H*-pyrido[1,2-a] pyrimidin-3-yl) methylene)-2-thioxothiazolidin-4-one |
| 3 | 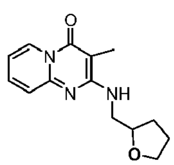 | 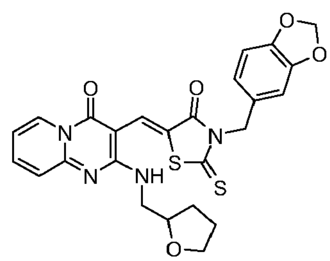 | (Z)-3-(benzo[d][1,3] dioxol-5-yl-methyl)-5-((4-oxo-2-((tetrahydrofuran-2-yl) methylamino)-4*H*-pyrido [1,2-a]pyrimidin-3-yl) methylene)-2-thioxothiazolidin-4-one |
| 4 | 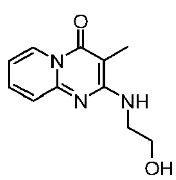 | 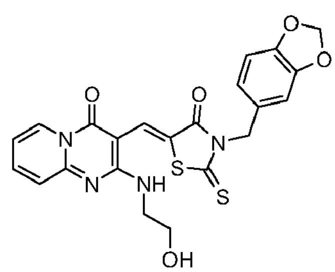 | (Z)-3-(benzo[d][1,3] dioxol-5-yl-methyl)-5-((2-(2-hydroxyethylamino)-4-oxo-4*H*-pyrido[1,2-a] pyrimidin-3-yl) methylene)-2-thioxothiazolidin-4-one |
| 5 | 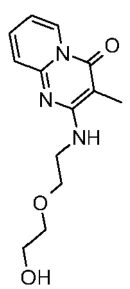 | 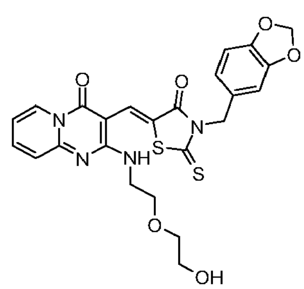 | (Z)-3-(benzo[d][1,3] dioxol-5-yl-methyl)-5-((2-(2-(2-hydroxyethoxy) ethylamino)-4-oxo-4*H*-pyrido[1,2-a] pyrimidin-3-yl) methylene)-2-thioxothiazolidin-4-one |
| 6 | 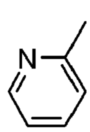 | 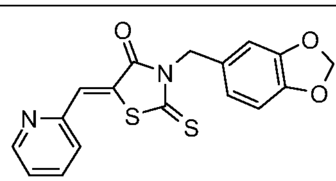 | (Z)-3-(benzo[d][1,3] dioxol-5-yl-methyl)-5-(pyridin-2-yl-methylene)-2-thioxothiazolidin-4-one |

(continued)

| Ex. No. | $R_1$ | Structure | Name |
|---|---|---|---|
| 7 | 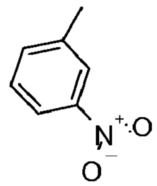 | 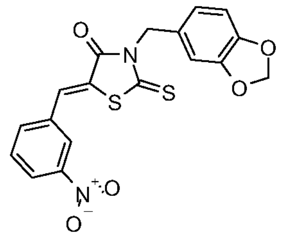 | (Z)-3-(benzo[d][1,3] dioxol-5-yl-methyl)-5-(3-nitrobenzylidene)-2-thioxothiazolidin-4-one |
| 8 | 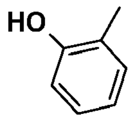 | 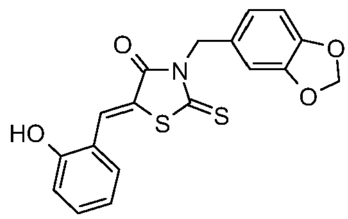 | (Z)-3-(benzo[d][1,3] dioxol-5-yl-methyl)-5-(2-hydroxybenzylidene)-2-thioxothiazolidin-4-one |
| 9 | 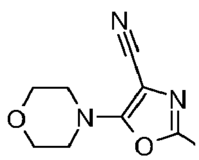 | 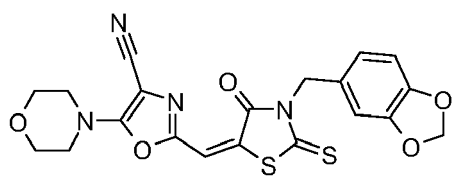 | (E)-2-((3-(benzo[d][1,3] dioxol-5-yl-methyl)-4-oxo-2-thioxothiazolidin-5-ylidene)methyl)-5-morpholinooxazole-4-carbonitrile |
| 10 | 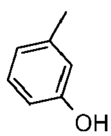 | 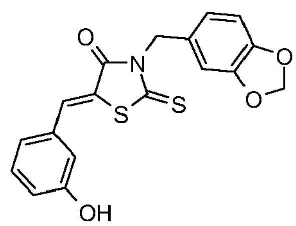 | (Z)-3-(benzo[d][1,3] dioxol-5-yl-methyl)-5-(3-hydroxybenzylidene)-2-thioxothiazolidin-4-one |
| 11 | 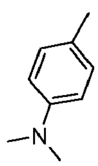 | 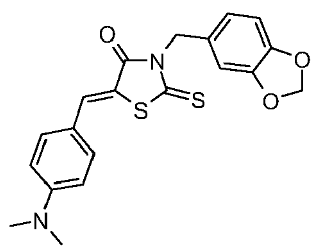 | (Z)-3-(benzo[d][1,3] dioxol-5-yl-methyl)-5-(4-(dimethylamino) benzylidene)-2-thioxothiazolidin-4-one |
| 12 | 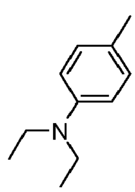 | 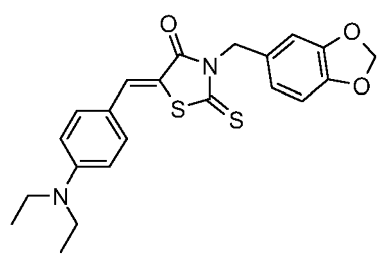 | (Z)-3-(benzo[d][1,3] dioxol-5-yl-methyl)-5-(4-(diethylamino) benzylidene)-2-thioxothiazolidin-4-one |

(continued)

| Ex. No. | $R_1$ | Structure | Name |
|---|---|---|---|
| 13 | 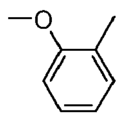 | 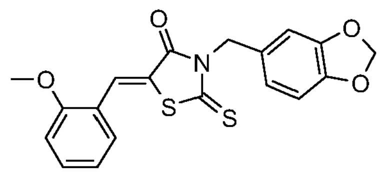 | (Z)-3-(benzo[d][1,3] dioxol-5-yl-methyl)-5-(2-methoxybenzylidene)-2-thioxothiazolidin-4-one |
| 14 | 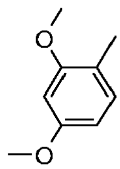 | 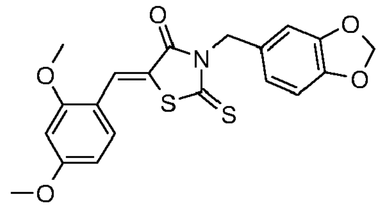 | (Z)-3-(benzo[d][1,3] dioxol-5-yl-methyl)-5-(2,4-dimethoxybenzylidene)-2-thioxothiazolidin-4-one |
| 15 | 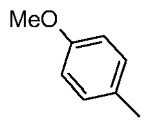 | 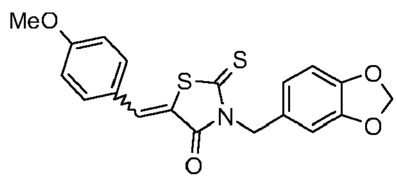 | 3-(benzo[d][1,3]dioxol-5-ylmethyl)-5-(4-methoxybenzylidene)-2-thioxothiazolidin-4-one |
| 16 | 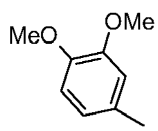 | 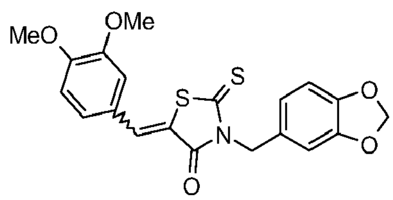 | 3-(benzo[d][1,3]dioxol-5-ylmethyl)-5-(3,4-dimethoxybenzylidene)-2-thioxothiazolidin-4-one |
| 17 | 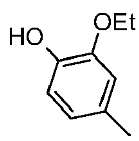 | 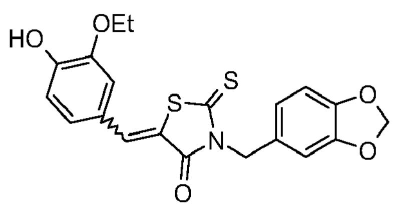 | 3-(benzo[d][1,3]dioxol-5-ylmethyl)-5-(3-ethoxy-4-hydroxybenzylidene)-2-thioxothiazolidin-4-one |
| 18 | 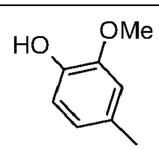 | 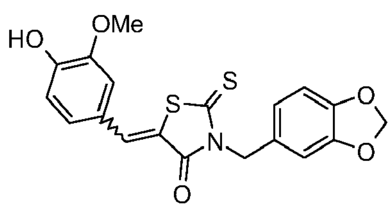 | 3-(benzo[d][1,3]dioxol-5-ylmethyl)-5-(4-hydroxy-3-methoxybenzylidene)-2-thioxothiazolidin-4-one |
| 19 | 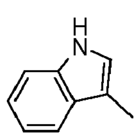 | 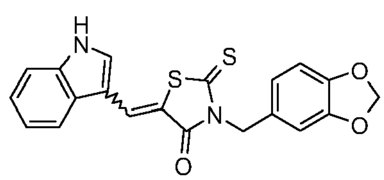 | 5-((1H-indol-3-yl) methylene)-3-(benzo[d] [1,3]dioxol-5-ylmethyl)-2-thioxothiazolidin-4-one |

(continued)

| Ex. No. | $R_1$ | Structure | Name |
|---|---|---|---|
| 20 | 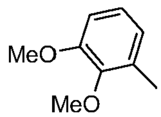 | 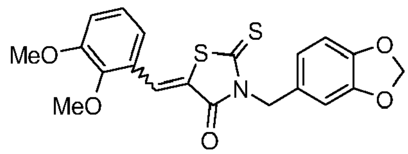 | 3-(benzo[d][1,3]dioxol-5-ylmethyl)-5-(2,3-dimethoxybenzylidene)-2-thioxothiazolidin-4-one |
| 21 | 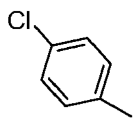 | 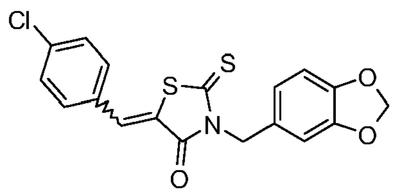 | 3-(benzo[d][1,3]dioxol-5-ylmethyl)-5-(4-chlorobenzylidene)-2-thioxothiazolidin-4-one |
| 22 | 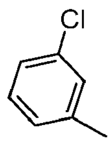 | 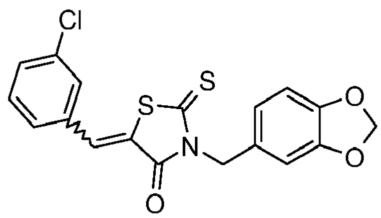 | 3-(benzo[d][1,3]dioxol-5-ylmethyl)-5-(3-chlorobenzylidene)-2-thioxothiazolidin-4-one |
| 23 | 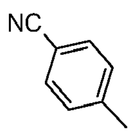 | 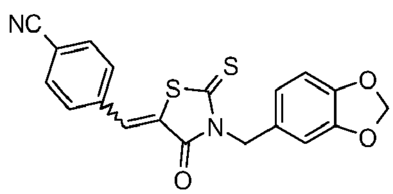 | 4-((3-(benzo[d][1,3]dioxol-5-ylmethyl)-4-oxo-2-thioxothiazolidin-5-ylidene)methyl)benzonitrile |
| 24 | 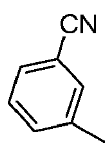 | 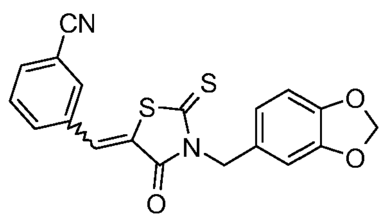 | 3-((3-(benzo[d][1,3]dioxol-5-ylmethyl)-4-oxo-2-thioxothiazolidin-5-ylidene)methyl)benzonitrile |
| 25 | 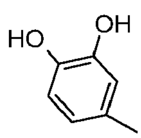 | 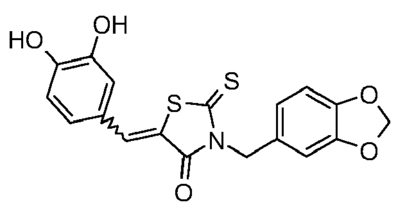 | 3-(benzo[d][1,3]dioxol-5-ylmethyl)-5-(3,4-dihydroxybenzylidene)-2-thioxothiazolidin-4-one |
| 26 | 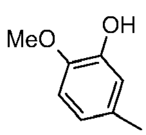 | 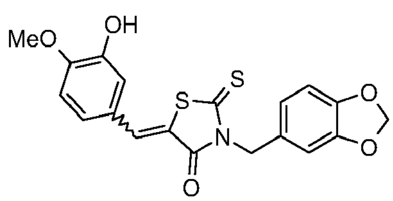 | 3-(benzo[d][1,3]dioxol-5-ylmethyl)-5-(3-hydroxy-4-methoxybenzylidene)-2-thioxothiazolidin-4-one |

(continued)

| Ex. No. | $R_1$ | Structure | Name |
|---|---|---|---|
| 27 | 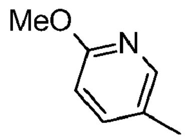 | 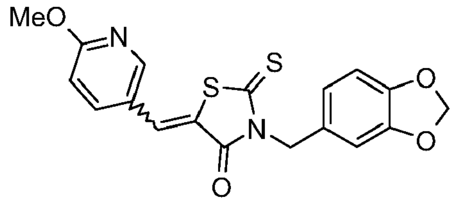 | 3-(benzo[d][1,3]dioxol-5-ylmethyl)-5-((6-methoxypyridin-3-yl) methylene)-2-thioxothiazolidin-4-one |
| 28 | 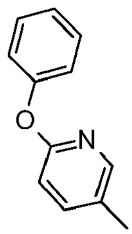 | 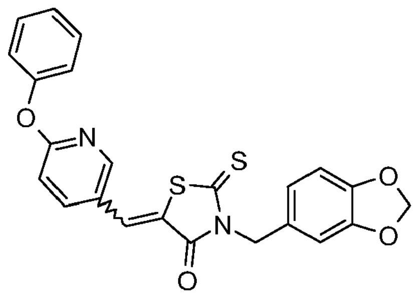 | 3-(benzo[d][1,3]dioxol-5-ylmethyl)-5-((6-phenoxypyridin-3-yl) methylene)-2-thioxothiazolidin-4-one |
| 29 | 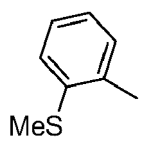 | 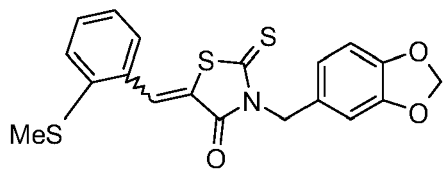 | 3-(benzo[d][1,3]dioxol-5-ylmethyl)-5-(2-(methylthio) benzylidene)-2-thioxothiazolidin-4-one |
| 30 | 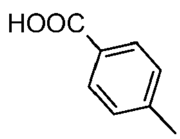 | 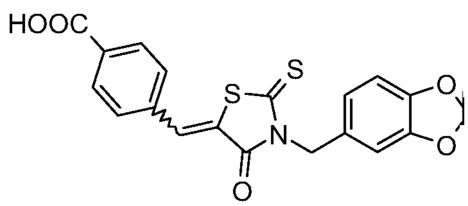 | 4-((3-(benzo[d][1,3] dioxol-5-ylmethyl)-4-oxo-2-thioxothiazolidin-5-ylidene)methyl) benzoic acid |
| 31 | 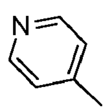 | 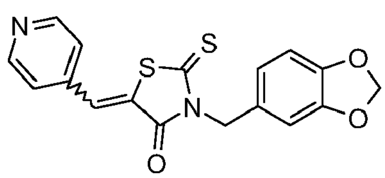 | 3-(benzo[d][1,3]dioxol-5-ylmethyl)-5-(pyridin-4-ylmethylene)-2-thioxothiazolidin-4-one |
| 32 | 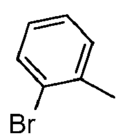 | 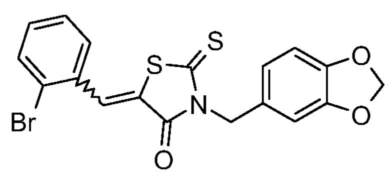 | 3-(benzo[d][1,3]dioxol-5-ylmethyl)-5-(2-bromobenzylidene)-2-thioxothiazolidin-4-one |
| 33 | 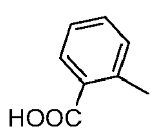 | 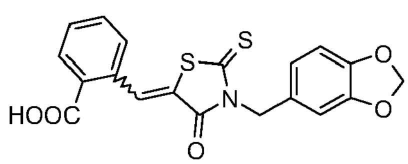 | 2-((3-(benzo[d][1,3] dioxol-5-ylmethyl)-4-oxo-2-thioxothiazolidin-5-ylidene)methyl) benzoic acid |

(continued)

| Ex. No. | $R_1$ | Structure | Name |
|---|---|---|---|
| 34 | 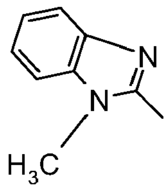 | 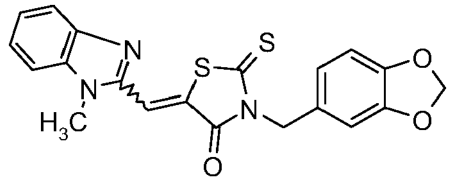 | 3-(benzo[d][1,3]dioxol-5-ylmethyl)-5-((1-methyl-1*H*-benzo[d]imidazol-2-yl)methylene)-2-thioxothiazolidin-4-one |
| 35 | 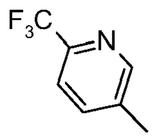 | 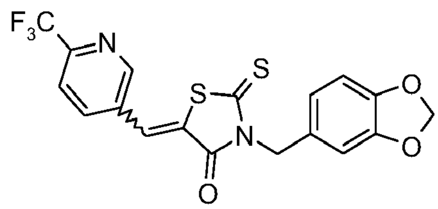 | 3-(benzo[d][1,3]dioxol-5-ylmethyl)-2-thioxo-5-((6-(trifluoromethyl)pyridin-3-yl)methylene)thiazolidin-4-one |
| 36 | 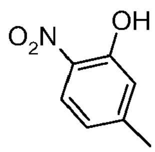 | 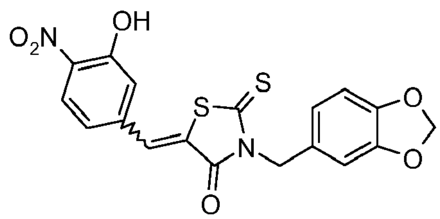 | 3-(benzo[d][1,3]dioxol-5-ylmethyl)-5-(3-hydroxy-4-nitrobenzylidene)-2-thioxothiazolidin-4-one |
| 37 | 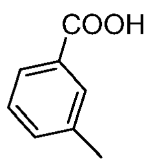 | 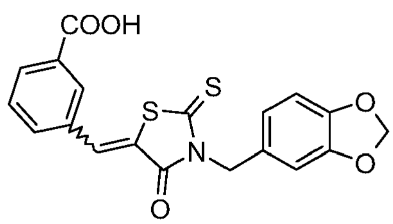 | 3-((3-(benzo[d][1,3]dioxol-5-ylmethyl)-4-oxo-2-thioxothiazolidin-5-ylidene)methyl)benzoic acid |
| 38 | 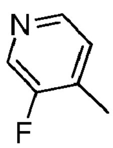 | 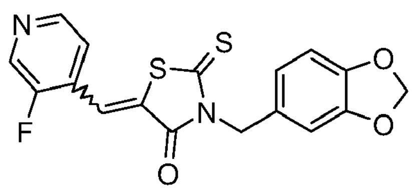 | 3-(benzo[d][1,3]dioxol-5-ylmethyl)-5-((3-fluoropyridin-4-yl)methylene)-2-thioxothiazolidin-4-one |
| 39 | 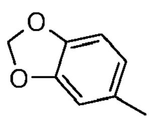 | 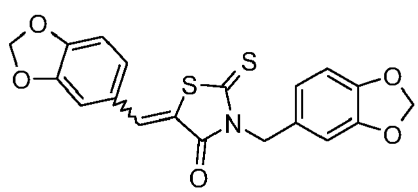 | 3-(benzo[d][1,3]dioxol-5-ylmethyl)-5-(benzo[d][1,3]dioxol-5-ylmethylene)-2-thioxothiazolidin-4-one |
| 40 | 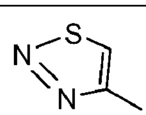 | 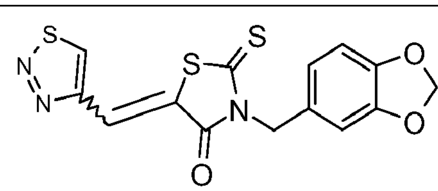 | 5-((1,2,3-thiadiazol-4-yl)methylene)-3-(benzo[d][1,3]dioxol-5-ylmethyl)-2-thioxothiazolidin-4-one |

(continued)

| Ex. No. | $R_1$ | Structure | Name |
|---|---|---|---|
| 41 | 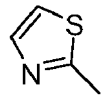 | 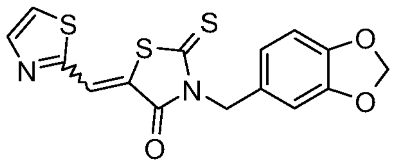 | 3-(benzo[d][1,3]dioxol-5-ylmethyl)-5-(thiazol-2-ylmethylene)-2-thioxothiazolidin-4-one |
| 42 | 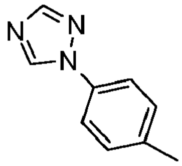 | 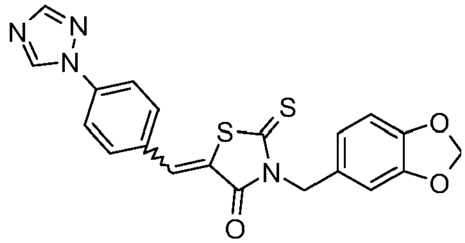 | 5-(4-(1*H*-1,2,4-triazol-1-yl)benzylidene)-3-(benzo[d][1,3]dioxol-5-ylmethyl)-2-thioxothiazolidin-4-one |
| 43 | 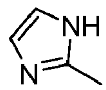 | 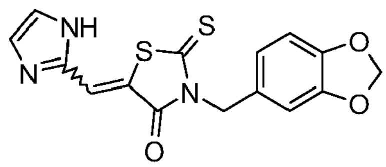 | 5(((1*H*-imidazol-2-yl) methylene)-3-(benzo[d] [1,3]dioxol-5-ylmethyl)-2-thioxothiazolidin-4-one |
| 44 | 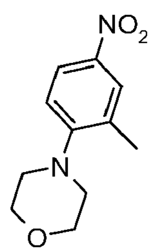 | 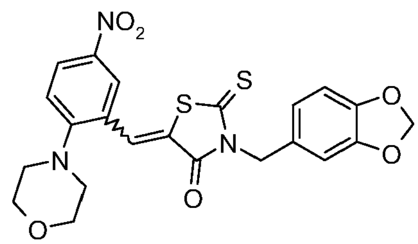 | 3-(benzo[d][1,3]dioxol-5-ylmethyl)-5-(2-morpholino-5-nitrobenzylidene)-2-thioxothiazolidin-4-one |
| 45 | 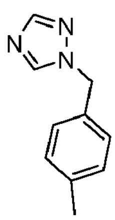 | 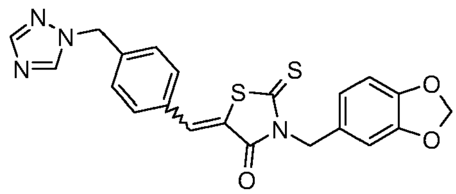 | 5-(4-((1*H*-1,2,4-triazol(1 (yl)methyl)benzylidene)-3-(benzo[d][1,3]dioxol-5-ylmethyl)-2-thioxothiazolidin-4-one |
| 46 | 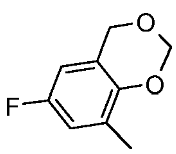 | 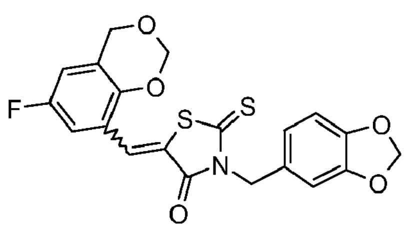 | 3-(benzo[d][1,3]dioxol-5-ylmethyl)-5-((6-fluoro-4*H*-(benzo[d][1,3]dioxin-8-yl)methylene)-2-thioxothiazolidin-4-one |

(continued)

| Ex. No. | $R_1$ | Structure | Name |
|---|---|---|---|
| 47 | 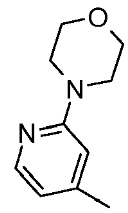 | 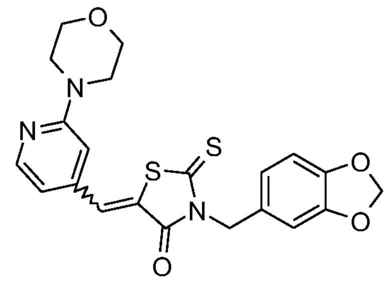 | 3-(benzo[d][1,3]dioxol-5-ylmethyl)-5-((2-morpholinopyridin-4-yl) methylene)-2-thioxothiazolidin-4-one |
| 48 | 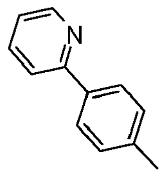 | 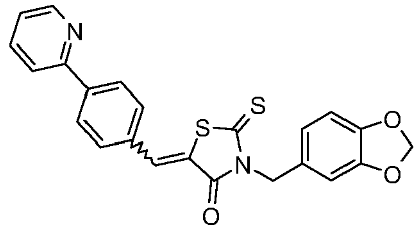 | 3-(benzo[d][1,3]dioxol-5-ylmethyl)-5-(4-(pyridin-2-yl) benzylidene)-2-thioxothiazolidin-4-one |
| 49 | 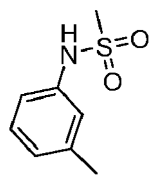 | 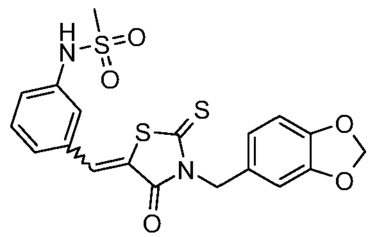 | *N*-(3-((3-(benzo[d][1,3] dioxol-5-ylmethyl)-4-oxo-2-thioxothiazolidin-5-ylidene)methyl) phenyl)methanesulfon amide |
| 50 | 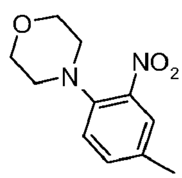 | 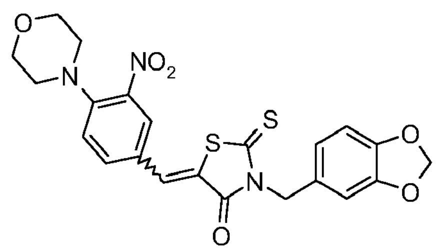 | 3-(benzo[d][1,3]dioxol-5-ylmethyl)-5-(4-morpholino-3-nitrobenzylidene)-2-thioxothiazolidin-4-one |
| 51 | 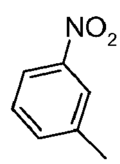 | 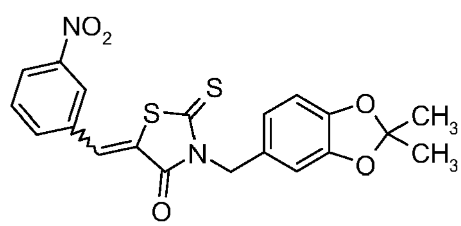 | 3-((2,2-dimethylbenzo [d][1,3]dioxol-5-yl) methyl)-5-(3-nitrobenzylidene)-2-thioxothiazolidin-4-one |
| 52 | 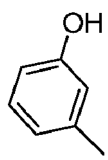 | 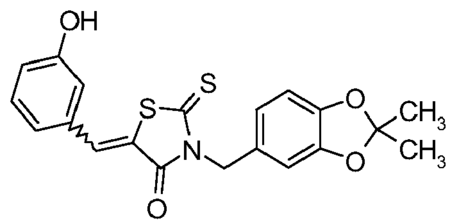 | 3-((2,2-dimethylbenzo [d][1,3]dioxol-5-yl) methyl)-5-(3-hydroxybenzylidene)-2-thioxothiazolidin-4-one |

(continued)

| Ex. No. | $R_1$ | Structure | Name |
|---|---|---|---|
| 53 | 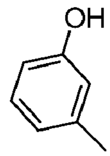 | 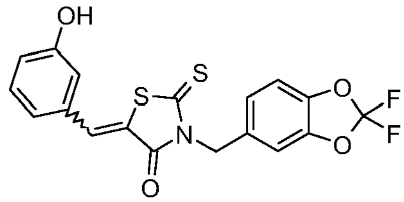 | 3-((2,2-difluorobenzo[d][1,3]dioxol-5-yl)methyl)-5-(3-hydroxybenzylidene)-2-thioxothiazolidin-4-one |
| 54 | 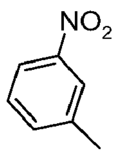 | 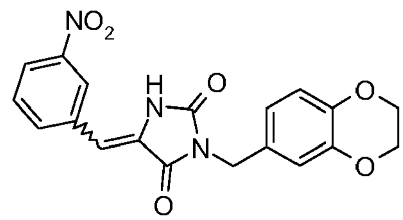 | 3-((2,3-dihydrobenzo[b][1,4]dioxin-6-yl)methyl)-5-(3-nitrobenzylidene) imidazolidine-2,4-dione |
| 55 | 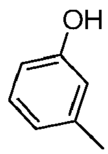 | 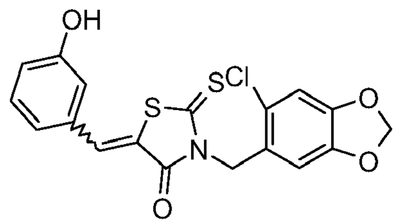 | 3-((6-chlorobenzo[d][1,3]dioxol-5-yl)methyl)-5-(3-hydroxybenzylidene)-2-thioxothiazolidin-4-one |
| 56 | 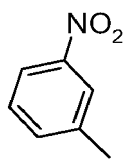 | 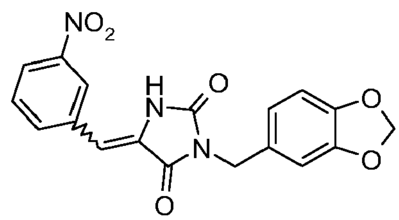 | 3-(benzo[d][1,3]dioxol-5-ylmethyl)-5-(3-nitrobenzylidene) imidazolidine-2,4-dione |
| 57 | 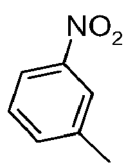 | 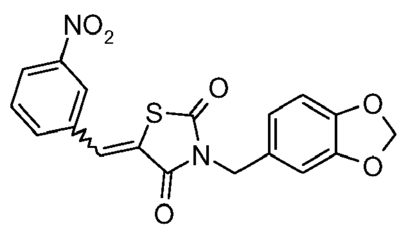 | 3-(benzo[d][1,3]dioxol-5-ylmethyl)-5-(3-nitrobenzylidene) thiazolidine-2,4-dione |
| 58 | 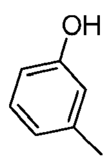 | 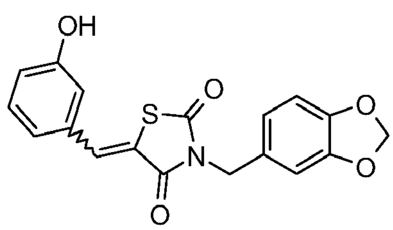 | 3-(benzo[d][1,3]dioxol-5-ylmethyl)-5-(3-hydroxybenzylidene) thiazolidine-2,4-dione |
| 59 | 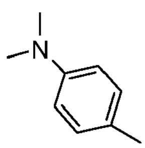 | 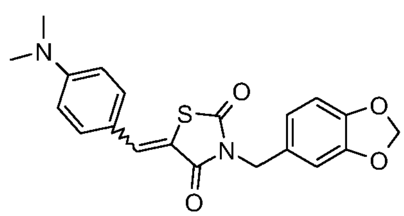 | 3-(benzo[d][1,3]dioxol-5-ylmethyl)-5-(4-(dimethylamino) benzylidene)thiazolidi ne-2,4-dione |

(continued)

| Ex. No. | $R_1$ | Structure | Name |
|---|---|---|---|
| 60 | 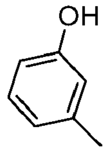 | 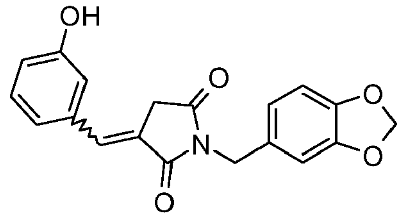 | 1-(benzo[d][1,3]dioxol-5-ylmethyl)-3-(3-hydroxybenzylidene) pyrrolidine-2,5-dione |
| 61 | 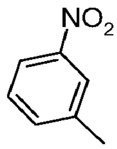 | 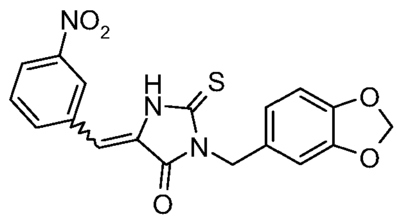 | 3-(benzo[d][1,3]dioxol-5-ylmethyl)-5-(3-nitrobenzylidene)-2-thioxoimidazolidin-4-one |

Analytical data for abovementioned examples:

Example 1:
1 H NMR (300MHz, DMSO-d6) δ 1.76 (2H, q, J = 6.5 Hz), 3.49 (2H, m), 3.55 (2H, m), 4.52 (1 H, t, J = 5.1 Hz), 5.13 (2H, s), 5.99 (2H, s), 6.80-7.00 (3H, m), 7.13 (1H, tm, J = 6.9 Hz), 7.34 (1H, d, J = 8.9), 7.74 (1H, s), 7.89 (1 H, m), 8.16 (1H, t, J = 5.4 Hz), ), 8.76 (1H, d, J = 7.1 Hz); EI MS m/z 496 [M+], FAB MS m/z 497 [MH+]

Example 2:
1 H NMR (300MHz, DMSO-d6) δ 2.97 (2H, d, J = 4.4), 5.14 (2H, s), 5.99 (2H, s), 6.80-6.96 (3H, m), 7.14 (1 H, tm, J = 6.9 Hz), 7.37 (1H, d, J = 8.9), 7.73 (1H, s), 7.90 (1H, m), 8.15 (1H, m), 8.78 (1H, d, J = 7.3 Hz); EI MS m/z 452 [M+], FAB MS m/z 453 [MH+]

Example 3:
1 H NMR (300MHz, DMSO-d6) δ 1.63 (1 H, m), 1.86 (3H, m), 3.44-3.70 (3H, m), 3.80 (1 H, m), 4.10 (1H, m), 5.14 (2H, s), 5.99 (2H, s), 6.80-7.00 (3H, m), 7.14 (1H, tm, J = 7.0 Hz), 7.34 (1 H, d, J = 8.8), 7.75 (1 H, s), 7.89 (1H, m), 8.28 (1 H, t, J = 5.5), 8.77 (1 H, d, J = 7.0 Hz); EI MS m/z 522 [M+], FAB MS m/z 523 [MH+]

Example 4:
1 H NMR (300MHz, DMSO-d6) δ 3.58 (4H, m), 4.78 (1H, m), 5.14 (2H, s), 5.99 (2H, s), 6.80-6.96 (3H, m), 7.13 (1H, tm, J = 6.9 Hz), 7.34 (1H, d, J = 8.9), 7.76 (1 H, s), 7.89 (1H, m), 8.15 (1 H, m), 8.77 (1 H, dm, J = 7.0 Hz); EI MS m/z 482 [M+], FAB MS m/z 483 [MH+]

Example 5:
1 H NMR (300MHz, DMSO-d6) δ 3.42-3.75 (8H, m), 4.59 (1H, m), 5.14 (2H, s), 5.99 (2H, s), 6.80-6.96 (3H, m), 7.14 (1H, td, J = 7.0 Hz, 1.1 Hz), 7.36 (1H, d, J = 8.8), 7.74 (1H, s), 7.90 (1 H, m), 8.22 (1 H, t, J = 5.0 Hz), 8.78 (1 H, d, J = 7.1 Hz); EI MS m/z 526 [M+], FAB MS m/z 527 [MH+]

Example 6:
1 H NMR (300MHz, DMSO-d6) δ 5.15 (2H, s), 5.99 (2H, s), 6.79-6.95 (3H, m), 7.46 (1 H, m), 7.87 (1H, s), 7.97 (2H, m), 8.81 (1H, d, J = 4.5 Hz); EI MS m/z 356 [M+], FAB MS m/z 357 [MH+]

Example 7:
1H NMR (300MHz, DMSO-d6) δ 5.16 (2H, s), 6.00 (2H, s), 6.80-6.98 (3H, m), 7.85 (1 H, t, J = 8.0 Hz), 8.03 (1H, s), 8.06 (1H, dm, J = 8.1 Hz), 8.33 (1H, dm, J = 8.2 Hz), 8.52 (1 H, m); EI MS m/z 400 [M+], FAB MS m/z 401 [MH+]

Example 8:
1H NMR (300MHz, DMSO-d6) δ 5.13 (2H, s), 5.98 (2H, s), 6.78-7.02 (5H, m), 7.30-7.40 (2H, m), 8.01 (1H, s), 10.77 (1H, s); EI MS m/z 371 [M+], FAB MS m/z 372 [MH+]

Example 9:
1H NMR (300MHz, DMSO-d6) δ 3.60 (4H, m), 3.77 (4H, m), 5.11 (2H, s), 5.99 (2H, s), 6.78-6.89 (2H, m), 6.91 (1H, s), 6.78-6.94 (3H, m), 7.32 (1 H, s); EI MS m/z 456 [M+], FAB MS m/z 457 [MH+]

Example 10:
1H NMR (300MHz, DMSO-d6) δ 5.14 (2H, s), 5.99 (2H, s), 6.80-6.96 (4H, m), 7.02 (1H, t, J = 1.9 Hz), 7.10 (1H, d, J =7.9 Hz), 7.36 (1H, d, J = 7.9 Hz), 7.75 (1H, s), 9.90 (1H, s); EI MS m/z 371 [M+], FAB MS m/z 372 [MH+]

Example 11:
1H NMR (300MHz, DMSO-d6) δ 3.04 (6H, s), 5.12 (2H, s), 5.98 (2H, s), 6.75-6.95 (5H, m), 7.48 (2H, m), 7.71 (1 H, s); EI MS m/z 398 [M+], FAB MS m/z 399 [MH+]

Example 12:
1H NMR (300MHz, DMSO-d6) δ 1.13 (6H, t, J = 7.0 Hz), 3.45 (4H, q, J = 7.1 Hz), 5.13 (2H, s), 5.99 (2H, s), 6.78-6.94 (5H, m), 7.46 (2H, m), 7.69 (1 H, s); FAB MS m/z 427 [MH+]

Example 13:
1H NMR (300MHz, DMSO-d6) δ 3.91 (3H, s), 5.14 (2H, s), 5.99 (2H, s), 6.80-6.89 (2H, m), 6.92 (1H, d, J = 1.3 Hz), 7.12 (1H, t, J = 7.5 Hz), 7.18 (1H, d, J = 8.4 Hz), 7.46 (1H, dd, J = 7.8 Hz, 1.5 Hz), 7.53 (1 H, m), 7.79 (1H, s); FAB MS m/z 386 [MH+]

Example 14:
1H NMR (300MHz, DMSO-d6) δ 3.86 (3H, s), 3.92 (3H, s), 5.13 (2H, s), 5.99 (2H, s), 6.68-6.93 (5H, m), 7.41 (1H, d, J = 8.5 Hz), 7.92 (1 H, s); FAB MS m/z 416 [MH+]

Example 15:
1H NMR (300 MHz, DMSO-d6) δ 3.83 (s, 3H), 5.13 (s, 2H), 5.98 (s, 2H), 6.81 (dd, 1H, J = 8.0 Hz, 1.4 Hz), 6.85 (d, 1H, J = 8.0 Hz), 6.90 (d, 1 H, J = 1.4 Hz), 7.12 (d, 2H, J = 9.0 Hz), 7.62 (d, 2H, J = 9.0 Hz), 7.81 (s, 1H); EI MS m/z 385 [M+].

Example 16:
1H NMR (300 MHz, DMSO-d6) δ 3.82 (s, 3H), 3.84 (s, 3H), 5.13 (s, 2H), 5.98 (s, 2H), 6.81 (dd, 1 H, J = 8.0 Hz, 1.3 Hz), 6.86 (d, 1H, J = 8.0 Hz), 6.91 (d, 1 H, J = 1.3 Hz), 7.21 (d, 1 H, J = 8.4 Hz), 7.22 (dd, 1 H, J1 = 5.7 Hz, 1.9 Hz), 7.26 (d, 1 H, J = 2.0 Hz), 7.80 (s, 1H). EI MS m/z 415 [M+].

Example 17:
1H NMR (300 MHz, DMSO-d6) δ 1.37 ( t, 3H, J = 6.9 Hz), 4.10 (q, 2H), 5.14 (s, 2H), 5.99 (s, 2H), 6.82 (dd, 1H, J1 = 8.0 Hz, 1.4 Hz), 6.86 (d, 1H, J = 8.0 Hz), 6.91 (d, 1H, J = 1.4 Hz), 6.97 (d, 1 H, J = 8.4 Hz), 7.14 (dd, 1 H, J = 8.4 Hz, 1.8 Hz), 7.18 (d, 1H, J = 1.8 Hz), 7.76 (s, 1 H), 10.08 (s, 1H). EI MS m/z 415 [M+].

Example 18:
1H NMR (300 MHz, DMSO-d6) δ 3.85 (s, 3H), 5.15 (s, 2H), 5.99 (s, 2H), 6.82 (dd, 1H, J = 8.1 Hz, 1.4 Hz), 6.87 (d, 1 H, J = 8.1 Hz), 6.92 ( d, 1 H, J= 1.4 Hz), 6.96 (d, 1 H, J = 8.3 Hz), 7.15 (dd, 1 H, J = 8.3 Hz, 2.0 Hz), 7.21 (d, 1 H, J= 2.0 Hz), 7.77 (s, 1 H), 10.15 (s, 1 H). EI MS m/z 401.

Example 19:
1H NMR (300 MHz, DMSO-d6) δ 5.16 (s, 2H), 5.99 (s, 2H), 6.81 - 6.94 (m, 3H), 7.21 -7.30 (m, 2H), 7.52 (d, 1 H, J = 7.6 Hz), 7.92 - 7.98 (m, 2H), 8.13 (s, 1 H), 12.39 (s, 1 H). EI MS m/z 394 [M+].

Example 20:
1H NMR (300 MHz, DMSO-d6) δ 3.82 (s, 3H), 3.87 (s, 3H), 5.15 (s, 2H), 6.00 (s, 2H), 6.84 (dd, J = 8.2 Hz, 1.0 Hz), 6.88 (d, 1 H, J = 8.2 Hz), 6.94 (d, 1 H, J = 1.0 Hz), 7.06 - 7.09 (m, 1H), 7.25 (d, 1H, J = 2.1 Hz), 7.27 (s, 1H), 7.92 (s, 1 H). FAB MS m/z 416 [M+1].

Example 21:
1H NMR (300 MHz, DMSO-d6) δ 5.15 (s, 2H), 5.99 (s, 2H), 6.83 (dd, 1 H, J = 8.0 Hz, 1.4 Hz), 6.87 (d, 1 H, J = 8.0 Hz), 6.92 (d, 1 H, J = 1.4 Hz), 7.62 (dd, 2H, J = 6.6 Hz, 2.2 Hz), 7.68 (dd, 2H, J = 6.6 Hz, 2.2 Hz), 7.85 (s, 1 H). EI MS m/z 389 [M+].

Example 22:
1H NMR (300 MHz, DMSO-d6) δ 5.15 (s, 2H), 6.00 (s, 2H), 6.84 (dd, 1 H, J = 7.9 Hz, 1.5 Hz), 6.88 (d, 1 H, J = 7.9 Hz), 6.93 (d, 1 H, J = 1.5 Hz), 7.57 - 7.59 (m, 3H), 7.75 (s, 1 H), 7.85 (s, 1 H). EI MS m/z 389 [M+].

Example 23:
1H NMR (300 MHz, DMSO-d6) δ 5.15 (s, 2H), 6.00 (s, 2H), 6.83 (dd, 1 H, J = 7.9 Hz, 1.2 Hz), 6.88 (d, 1 H, J = 7.9 Hz), 6.93 (d, 1 H, J = 1.2 Hz), 7.83 (d, 2H, J = 8.4 Hz), 7.91 (s, 1 H), 8.01 (d, 2H, J = 8.4 Hz). FAB MS m/z 380 [M+1].

Example 24:
1H NMR (300 MHz, DMSO-d6) δ 5.16 (s, 2H), 6.00 (s, 2H), 6.83 - 6.93 (m, 3H), 7.74 - 7.79 (m, 1 H), 7.88 - 7.99 (m, 3H), 8.15 (s, 1 H). EI MS m/z 380 [M+].

Example 25:
1H NMR (300 MHz, DMSO-d6) δ 5.13 (s, 2H), 5.99 (s, 2H), 6.82 (dd, 1 H, J = 8.1 Hz, 1.5 Hz), 6.85 - 6.88 (m, 3H), 6.91 (s, 1 H), 7.04 - 7.11 (m, 2H), 7.67 (s, 1 H), 9.80 (bs, 2H). EI MS m/z 387 [M+].

Example 26:
1H NMR (300 MHz, DMSO-d6) δ 3.85 (s, 3H), 5.14 (s, 2H), 5.99 (s, 2H), 6.83 (dd, 1 H, J = 7.9 Hz, 1.7 Hz), 6.87 (d, 1H, J = 7.9 Hz), 6.92 (m, 1H), 7.07 (d, 1H, J = 2.2 Hz), 7.10 (d, 1H, J = 8.5 Hz), 7.17 (dd, 1 H, J = 8.5 Hz, 2.2 Hz), 7.71 (s, 1 H), 9.59 (s, 1 H). EI MS m/z 401 [M+].

Example 27:
1H NMR (300 MHz, DMSO-d6) δ 3.94 (s, 3H), 5.15 (s, 2H), 5.99 (s, 2H), 6.83 (d, 1 H, J = 8.0 Hz), 6.87 (d, 1 H, J = 8.0 Hz), 6.92 (s, 1 H), 7.02 (d, 1 H, J = 8.8 Hz), 7.86 (s, 1 H), 7.93 (dd, 1 H, J = 8.8 Hz, 2.3 Hz), 8.57 (d, 1 H, J = 2.3 Hz). FAB MS m/z 387 [M+1].

Example 28:
1H NMR (300 MHz, DMSO-d6) δ 5.15 (s, 2H), 5.99 (s, 2H), 6.82 - 6.92 (m, 3H), 7.19 - 7.25 (m, 3H), 7.27 - 7.30 (m, 1 H), 7.43 - 7.49 (m, 2H), 7.86 (s, 1 H), 8.06 (dd, 1 H, J = 8.7 Hz, 2.0 Hz), 8.50 (d, 1 H, J = 2.0 Hz). EI MS m/z 448 [M+].

Example 29:
1H NMR (300 MHz, DMSO-d6) δ 2.56 (s, 3H), 5.14 (s, 2H), 6.00 (s, 2H), 6.81 - 6.94 (m, 3H), 7.33 - 7.38 (m, 1 H), 7.45 - 7.51 (m, 3H), 7.99 (s, 1 H). EI MS m/z 401 [M+].

Example 30:
1H NMR (300 MHz, DMSO-d6) δ 5.15 (s, 2H), 5.99 (s, 2H), 6.84 (dd, 1 H, J = 8.0 Hz, 1.1 Hz), 6.87 (d, 1 H, J = 8.9 Hz), 6.93 ( d, 1 H, J = 1.1 Hz), 7.71 (d, 2H, J = 8.4 Hz), 7.88 (s, 1 H), 8.05 (d, 2H, J = 8.4 Hz). EI MS m/z 399 [M+].

Example 31:
1H NMR (300 MHz, DMSO-d6) δ 5.15 (s, 2H), 5.99 (s, 2H), 6.84 (dd, 1 H, J = 8.0 Hz, 1.4 Hz), 6.87 (d, 1 H, J = 8.0 Hz), 6.93 (d, 1 H, J= 1.4 Hz), 7.57 (dd, 2H, J = 4.6 Hz, 1.6 Hz), 7.81 (s, 1 H), 8.75 (dd, J = 4.6 Hz, 1.6 Hz). EI MS m/z 356 [M+].

Example 32:
1H NMR (300 MHz, DMSO-d6) δ 5.15 (s, 2H), 6.00 (s, 2H), 6.84 - 6.94 (m, 3H), 7.42 - 7.48 (m, 1 H), 7.59 (d, 2H, J = 4.2 Hz), 7.84 (d, 1 H, J = 7.8 Hz), 7.90 (s, 1 H). EI MS m/z 433 [M+].

Example 33:
1H NMR (300 MHz, DMSO-d6) δ 5.15 (s, 2H), 6.00 (s, 2H), 6.81 - 6.94 (m, 3H), 7.59 - 7.63 (m, 2H), 7.71 - 7.78 (m, 1 H), 8.02 (d, 1 H, J = 7.8 Hz), 8.46 (s, 1 H). EI MS m/z 399 [M+].

Example 34:
1H NMR (300 MHz, DMSO-d6) δ 4.03 (s, 3H), 5.18 (s, 2H), 6.00 (s, 2H), 6.85 (dd, 1 H, J = 7.9 Hz, 1.5 Hz), 6.88 (d, 1 H, J = 7.9 Hz), 6.94 (d, 1 H, J = 1.5 Hz), 7.30 - 7.43 (m, 2H), 7.69 (d, 1 H, J = 7.9 Hz), 7.80 (d, 1 H, J = 7.9 Hz), 7.88 (s, 1 H). EI MS m/z 409 [M+].

Example 35:
1H NMR (300 MHz, DMSO-d6) δ 5.16 (s, 2H), 6.00 (s, 2H), 6.84 (dd, 1 H, J = 7.9 Hz, 1.2 Hz), 6.88 (d, 1 H, J = 7.9

Hz), 6.93 ( d, 1 H, J = 1.2 Hz), 7.97 (s, 1 H), 8.06 (d, 1 H, J = 8.1 Hz), 8.27 (dd, 1 H, J = 8.1 Hz, 1.8 Hz), 9.01 (d, 1 H, J = 1.8 Hz). EI MS m/z 424 [M+].

Example 36:
1H NMR (300 MHz, DMSO-d6) δ 5.13 (s, 2H), 5.99 (s, 2H), 6.82 (dd, 1 H, J = 8.0 Hz, 1.1 Hz), 6.86 (d, 1 H, J = 8.0 Hz), 6.92 ( d, 1 H, J = 1.1 Hz), 6.98 (dd, 1 H, J = 8.6 Hz, 1.8 Hz), 7.20 (d, 1H, J = 1.8 Hz), 7.72 (s, 1H), 7.93 (d, 1H, J = 8.6 Hz). EI MS m/z 416 [M+].

Example 37:
1H NMR (300 MHz, DMSO-d6) δ 5.16 (s, 2H), 6.00 (s, 2H), 6.79 - 6.93 (m, 3H), 7.68 (t, 1 H, J = 7.7 Hz), 7.89 (d, 1 H, J = 8.1 Hz), 7.93 (s, 1 H), 8.05 (d, 1 H, J = 7.5 Hz), 8.18 (s, 1 H). EI MS 399 [M+].

Example 38:
1H NMR (300 MHz, DMSO-d6) δ 5.15 (s, 2H), 6.00 (s, 2H), 6.83 - 6.93 (m, 3H), 7.58 (t, 1 H, J = 5.7 Hz), 7.72 (s, 1 H), 8.61 (d, 1 H, J = 4.8 Hz), 8.77 (d, 1 H, J = 2.1 Hz). EI MS m/z 374 [M+].

Example 39:
1H NMR (300 MHz, DMSO-d6) δ 5.14 (s, 2H), 5.99 (s, 2H), 6.16 (s, 2H), 6.81 - 6.92 (m, 3H), 7.10 - 7.24 (m, 3H), 7.78 (s, 1 H). EI MS m/z 399 [M+].

Example 40:
1H NMR (300 MHz, DMSO-d6) δ 5.16 (s, 2H), 6.00 (s, 2H), 6.83 - 6.94 (m, 3H), 8.12 (s, 1 H), 9.70 (s, 1 H). EI MS m/z 363 [M+].

Example 41:
1H NMR (300 MHz, DMSO-d6) δ 5.14 (s, 2H), 5.99 (s, 2H), 6.82 (dd, 1 H, J = 8.0 Hz, 1.4 Hz), 6.87 (d, 1 H, J = 8.0 Hz), 6.92 (d, 1 H, J = 1.4 Hz), 8.09 (s, 1 H), 8.11 (d, 1 H, J = 3.0 Hz), 8.24 (d, 1 H, J = 3.0 Hz). EI MS m/z 362 [M+].

Example 42:
1H NMR (300 MHz, DMSO-d6) δ 5.17 (s, 2H), 6.00 (s, 2H), 6.84 (dd, 1 H, J = 7.9 Hz, 1.0 Hz), 6.88 (d, 1 H, J = 7.9 Hz), 6.94 (d, 1 H, J = 1.0 Hz), 7.86 (d, 2H, J = 8.9 Hz), 7.91 (s, 1 H), 8.07 (d, 2H, J = 8.9 Hz), 8.31 (s, 1 H), 9.43 (s, 1 H). EI MS m/z 422 [M+].

Example 43:
1H NMR (300 MHz, DMSO-d6) δ 5.12 (s, 2H), 5.99 (s, 2H), 6.82 (dd, 1 H, J = 7.9 Hz, 1.5 Hz), 6.86 (d, 1 H, J = 7.9 Hz), 6.91 (d, 1 H, J = 1.5 Hz), 7.39 (s, 1 H), 7.51 (s, 1 H), 7.53 (s, 1 H), 12.97 (s, 1 H). EI MS m/z 345 [M+].

Example 44:
1H NMR (300 MHz, DMSO-d6) δ 3.18 (t, 4H, J = 4.4 Hz), 3.77 (t, 4H, J = 4.2 Hz), 5.15 (s, 2H), 6.00 (s, 2H), 6.87 - 6.95 (m, 3H), 7.33 (d, 1 H, J = 7.3 Hz), 7.72 (s, 1 H), 8.26 (d, 1 H, J = 2.7 Hz), 8.30 (dd, 1 H, J = 7.3 Hz, 2.7 Hz). EI MS m/z 485 [M+].

Example 45:
1H NMR (300 MHz, DMSO-d6) δ 5.14 (s, 2H), 5.51 (s, 2H), 5.99 (s, 2H), 6.83 (dd, 1 H, J = 8.0 Hz, 1.3 Hz), 6.86 (d, 1 H, J = 8.0 Hz), 6.92 (d, 1 H, J = 1.3 Hz), 7.42 (d, 2H, J = 8.3 Hz), 7.64 (d, 2H, J = 8.3 Hz), 7.82 (s, 1 H), 8.01 (s, 1 H), 8.69 (s, 1 H). EI MS m/z 436 [M+].

Example 46:
1H NMR (300 MHz, DMSO-d6) δ 4.99 (s, 2H), 5.14 (s, 2H), 5.41 (s, 2H), 5.99 (s, 2H), 6.83 (d, 1 H, J = 8.0 Hz), 6.87 (d, 1 H, J = 8.0 Hz), 6.92 (s, 1 H), 7.16 (d, 1 H, J = 9.0 Hz), 7.20 (d, 1 H, J = 9.0 Hz), 7.82 (s, 1 H). EI MS m/z 431 [M+].

Example 47:
1H NMR (300 MHz, DMSO-d6) δ 3.50 (t, 4H, J = 4.8 Hz), 3.72 (t, 4H, J = 4.8 Hz), 5.15 (s, 2H), 6.00 (s, 2H), 6.81 (dd, 1 H, J = 6.6 Hz, 1.2 Hz), 6.84 (d, 1 H, J = 1.2 Hz), 6.87 (d, 1 H, J = 6.6 Hz), 6.92 (d, 1 H, J = 1.3 Hz), 7.04 (s, 1 H), 7.72 (s, 1 H), 8.28 (d, 1 H, J = 5.1 Hz). EI MS m/z 441 [M+].

Example 48:
1H NMR (300 MHz, DMSO-d6) δ 5.16 (s, 2H), 6.00 (s, 2H), 6.85 (dd, 1 H, J = 7.9 Hz, 1.4 Hz), 6.88 (d, 1 H, J = 7.9

Hz), 6.94 (d, 1 H, J = 1.4 Hz), 7.39 - 7.43 (m, 1 H), 7.77 (d, 2H, J = 8.6 Hz), 7.90 (s, 1 H), 7.94 (dd, 1 H, J = 7.7 Hz, 1.8 Hz), 8.07 (d, 1 H, J = 8.1 Hz), 8.28 (d, 2H, J = 8.6 Hz), 8.70 - 8.72 (m, 1 H). EI MS m/z 432 [M+].

Example 49:
1H NMR (300 MHz, DMSO-d6) δ 3.07 (s, 3H), 5.15 (s, 2H), 5.99 (s, 2H), 6.83 (dd, 1 H, J = 8.0 Hz, 1.1 Hz), 6.87 (d, 1 H, J = 8.0 Hz), 6.93 (d, 1 H, J = 1.1 Hz), 7.32 (d, 1 H, J = 7.8 Hz), 7.40 (d, 1 H, J = 8.1 Hz), 7.45 (s, 1 H), 7.51 (t, 1 H, J = 8.0 Hz), 7.80 (s, 1 H), 10.35 (bs, 1 H). EI MS m/z 448 [M+].

Example 50:
1H NMR (300 MHz, DMSO-d6) δ (ppm) 3.18 (t, 4H, J = 4.4 Hz), 3.71 (t, 4H, J = 4.4 Hz), 5.15 (s, 2H), 6.00 (s, 2H), 6.85 - 6.93 (m, 3H), 7.42 (d, 1 H, J = 9 Hz), 7.77 (dd, 1 H, J = 9 Hz, 2.3 Hz), 7.85 (s, 1H), 8.17 (d, 1H, J = 2.3 Hz). EI MS m/z 485 [M+].

Example 51:
1H NMR (300 MHz, DMSO-d6) δ (ppm) 1.60 (s, 6H), 5.13 (s, 2H), 6.75-6.84 (m, 3H), 7.81-7.86 (m, 1 H), 8.01 (s, 1 H), 8.03-8.06 (m, 1 H), 8.32 (ddd, 1 H, J = 8.2 Hz, 2.3 Hz, 0.9 Hz), 8.50-8.51 (m, 1 H). EI MS m/z 428 [M+].

Example 52:
1H NMR (300 MHz, DMSO-d6) δ (ppm) 1.60 (s, 6H), 5.11 (s, 2H), 6.74-6.84 (m, 3H), 6.91 (ddd, 1 H, J = 8.1 Hz, 2.38 Hz, 0.82 Hz), 7.00-7.02 (m, 1 H), 7.07-7.11 (m, 1 H), 7.32-7.37 (m, 1 H), 7.74 (s, 1 H). EI MS m/z 399 [M+].

Example 53:
1H NMR (300 MHz, DMSO-d6) δ (ppm) 5.23 (s, 2H), 6.92 (ddd, 1 H, J = 8.2 Hz, 2.4 Hz, 0.8 Hz), 7.01-7.03 (m, 1 H), 7.08-7.09 (m, 1 H), 7.13 (dd, 1 H, J = 8.4 Hz, 1.7 Hz), 7.33-7.41 (m, 3H), 7.75 (s, 1 H), 9.90 (s 1 H). EI MS m/z 407 [M+].

Example 54:
1H NMR (300 MHz, DMSO-d6) δ (ppm) 4.20 (s, 4H), 5.12 (s, 2H), 6.81-6.85 (m, 3H), 7.81-7.86 (m, 1 H), 8.01 - 8.06 (m, 2H), 8.32 (d, 1 H, J = 8.1 Hz), 8.50 (s 1 H). EI MS m/z 414 [M+].

Example 55:
1H NMR (300 MHz, DMSO-d6) δ (ppm) 4.80 (s, 2H), 6.07 (s, 2H), 6.89-7.11 (m, 5H), 7.32-7.38 (m, 1 H), 7.85 (s, 1 H), 9.87 (s, 1 H). EI MS m/z 418 [M+].

Example 56:
1H NMR (300 MHz, DMSO-d6) δ (ppm) 4.60 (s, 2H), 5.90-5.98 (m, 4H), 6.52 (d, 1H, J = 7.9 Hz), 6.84 (s, 1 H), 7.42 (d, 1 H, J = 7.8 Hz), 7.45-7.54 (m, 1 H), 7.83 (s, 1 H), 8.07 (bs, 1 H), 8.18-8.21 (m, 1 H). EI MS m/z 367 [M+].

Example 57:
1H NMR (300 MHz, DMSO-d6) δ (ppm) 4.74 (s, 2H), 5.99 (s, 2H), 6.81 (dd, 1 H, J = 8.1 Hz, 1.7 Hz), 6.86 (s, 1 H), 6.88 (d, 1 H, J = 1.8 Hz), 7.80-7.85 (m, 1 H), 8.02-8.04 (m, 1 H), 8.11 (s, 1 H), 8.30 (ddd, 1 H, J = 8.3 Hz, 2.3 Hz, 0.9 Hz), 8.47-8.48 (m, 1 H). EI MS m/z 384 [M+].

Example 58:
1H NMR (300 MHz, DMSO-d6) δ (ppm) 4.73 (s, 2H), 6.00 (s, 2H), 6.80 (dd, 1 H, J = 8.1 Hz, 1.6 Hz), 6.86-6.92 (m, 3H), 7.00-7.05 (m, 2H), 7.30-7.35 (m, 1 H), 7.85 (s, 1 H). ES+ MS m/z 356 [MH+].

Example 59:
1H NMR (300 MHz, DMSO-d6) δ (ppm) 3.01 (s, 6H), 4.70 (s, 2H), 5.98 (s, 2H), 6.78-6.87 (m, 5H), 7.45 (d, 2H, J = 8.9 Hz), 7.81 (s, 1 H). ES+ MS m/z 383 [MH+].

Example 60:
1H NMR (300 MHz, DMSO-d6) δ (ppm) 3.70 (s, 2H), 4.57 (s, 2H), 5.97 (s, 2H), 6.76-6.86 (m, 4H), 7.00 (s, 1 H), 7.06 (d, 1 H, J = 7.8), 7.24-7.29 (m, 1 H), 7.37 (s, 1 H), 9.66 (s, 1 H). EI MS m/z 337 [M+].

Example 61:
1H NMR (300 MHz, DMSO-d6) δ (ppm) 4.91 (s, 2H), 5.98 (s, 2H), 6.78-6.93 (m, 4H), 7.67-7.72 (m, 1H), 8.12 (d, 1H, J = 7.8 Hz), 8.19 (dd, 1H, J1 = 9.7 Hz, 1.53 Hz), 8.49 (s, 1H), 12.74 (bs, 1 H). EI MS m/z 383 [M+].

**Example 62: Sythesis of 3-((2,3-dihydrobenzo[b][1,4]dioxin-6-yl)methyl)-5-(3-nitrobenzylidene)imidazolidine-2,4-dione (compound of Example 54)**

**Step 1. Preparation of 3-((2,3-dihydrobenzo[b][1,4]dioxin-6-yl)methyl)-2-imidazolidin-4-one**

A suspension of (2,3-Dihydro-benzo[1,4]dioxin-6-yl)-methylamine (16.5 mmol, 2.73 g) in 1M NaOH is refluxed for 10 min and cooled to room temperature. Subsequently bis(carboxymethyl) trithiocarbonate (3.74 g, 16.5 mmol) is added and the reaction mixture refluxed overnight to give the precipitate, which is filtered off and is washed with water (3x 20 mL). The remaining residue is suspended in a mixture of methanol and isopropanol (1:1, 25 mL) and refluxed for 10 minutes. Cooled reaction mixture is filtered and the solid residue dried at lower temperature and recrystallyzed from methanol to give 3.29 g of 3-((2,3-dihydrobenzo[b][1,4]dioxin-6-yl)methyl)-imidazolidin-2,4-dione.

**Step 2. Coupling of aldehydes or ketones to 3-((2,3-dihydrobenzo[b][1,4]dioxin-6-yl)methyl)-imidazolidin-2,4-dione**

A solution of 74.5 mg (=0.3 mmol) 3-(benzo[d][1,3]dioxol-5-ylmethyl)-2-thioxothiazolidin-4-one, corresponding aldehyde R1 HCO (0.3 mmol) and sodium acetate (127.4 mg, 1.50 mmol) in acetic acid (3 mL) is heated in the microwave reactor for 2h at around 130˚C. The reaction mixture is cooled to room temperature and the obtained precipitate filtered off and washed with EtOH (2x5 mL) and MeOH (2x5mL) and dried at elevated temperature.

**Example 63: 3-(benzo[d][1,3]dioxol-5-ylmethyl)-5-(3-nitrobenzylidene)imidazo-lidine-2,4-dione**

**Step 1. Synthesis of 5-(3-nitrobenzylidene)imidazolidine-2,4-dione**

Coupling of aldehydes to imidazolidin-2,4-dione is performed in a similar manner as described in example 62.

**Step 2. Synthesis of 3-(benzo[d][1,3]dioxol-5-ylmethyl)-5-(3-nitrobenzylidene)imidazolidine-2,4-dione**

DBU (3.82 mmol, 0.961 g) is added into solution of 5-(3-nitrobenzylidene)imidazolidine-2,4-dione (3.19 mmol, 0.743 g) at 0˚C and the solution left to warm to room temperature. 5-(Chloromethyl)benzo[d][1,3]dioxole (6.37 mmol, 2.174 g, 50% soln in $CH_2Cl_2$) is added and the reaction mixture stirred for 3 hours. Subsequently 0.5 M HCL solution is added and extracted with ethylacetate (2 x 50 mL) dried over $MgSO_4$ and the solvent removed under reduced pressure. The obtained product is recrystallized from diisopropylether to afford 0.13 (11%) of 3-(benzo[d][1,3]dioxol-5-ylmethyl)-5-(3-nitrobenzylidene)imidazo-lidine-2,4-dione.

**Example 64: Synthesis of 3-(benzo[d][1,3]dioxol-5-ylmethyl)thiazolidine-2,4-dione**

A suspension of thiazolidine-2,4-dione (10.0 mmol, 1.17 g), 5-(chloromethyl)benzo[d][1,3]dioxole (10.0 mmol, 50% soln in $CH_2Cl_2$) and potassium fluoride (10.0 mmol, 0.581 g) is stirred at room temperature overnight. The solvent is removed under reduced pressure and the residue dissolved in $H_2O$ (50 mL) and extracted into dichloromethane (3 x 20 mL). Combined organic fractions were dried over $Na_2SO_4$ and the solvent removed under reduced pressure. The obtained oily product is washed with diethyl ether to give 1.590 g (64%) of 3-(benzo[d][1,3]dioxol-5-ylmethyl)thiazolidine-2,4-dione as a white precipitate.

**General Procedure D: Sythesis of 1-(benzo[d][1,3]dioxol-5-ylmethyl)-3-substituted-pyrrolidine-2,5-dione**

**Step 1. Synthesis of 1-(benzo[d][1,3]dioxol-5-ylmethyl)-1H-pyrrole-2,5-dione**

Diethylazodicarboxylate (20.0 mmol, 3.15 mL) is added into a solution of maleimide (20.0 mmol, 1.94 g), piperonyl alcohol (20.0 mmol, 3.043 g) and triphenylphosphine (20.0 mmol, 5.246 g) in TH F (100 mL) and stirred at room temperature for 1 hour. The solvent is removed under reduced pressure and the residue purified by column chromatography (silicagel; ethylacetatelhexane = ½) to yield 2.26 g (49%) of 1-(benzo[d][1,3]dioxol-5-ylmethyl)-1H-pyrrole-2,5-dione.

**Step 2. Coupling of aldehydes to 1-(benzo[d][1,3]dioxol-5-ylmethyl)-1H-pyrrole-2,5-dione**

A solution of 1-(benzo[d][1,3]dioxol-5-ylmethyl)-1H-pyrrole-2,5-dione (0.87 mmol, 0.20 g) and triphenylphosphine (0.87 mmol, 0.227 g) in acetic acid is heated for 1 hour at 110 ˚C and the solvent removed under reduced pressure.

The remaining oily residue is suspended in toluene (3 mL), a corresponding aldehyde (0.87 mmol) is added and heated the reaction mixture for 30 minutes at 100°C. The obtained precipitate is filtered off, washed with THF and dried at elevated temperature.

**Example 65: Synthesis of 3-(benzo[d][1,3]dioxol-5-ylmethyl)-2-thioxoimidazolidin-4-one**

A solution of glycine ethyl ester hydrochloride (2.0 mmol, 0.297 g), 5-(isothiocyanatomethyl)benzo[d][1,3]dioxole (2.00 mmol, 0.386 g) and sodium hydrogencarbonate (3.0 mmol, 0.252 g) in DMF (10 mL) is stirred at room temperature for 1 hour and then at 65°C overnight. The solvent is removed under reduced pressure and the oily residue purified by column chromatography (silicagel; ethylacetate/hexane= ½) to yield 0.339 g (68%) of 3-(benzo[d][1,3]dioxol-5-ylmethyl)-2-thioxoimidazolidin-4-one.

**Example 66. Enzyme Assays and $IC_{50}$-determination**

Method A: MurD enzyme assay

The compounds are tested for their ability to inhibit the formation of UDP-MurNAc-L-Ala-D-[$^{14}$C]Glu in mixtures (50 μl) containing 0.1 M Tris-HCl, pH 8.6, 5 mM $MgCl_2$, 5 mM ATP, 25 μM UDP-MurNAc-L-Ala, 25 μM D-[$^{14}$C]Glu, inhibitor (100 μM), DMSO (5%, v/v), and enzyme. After 30 min at 37°C, the reaction is stopped by addition of acetic acid (10 μl). After lyophilization, D-[$^{14}$C]Glu and of UDP-MurNAc-L-Ala-D-[$^{14}$C]Glu are separated by reverse-phase HPLC and quantitated by on-line scintillation counting. The residual activity is determined with respect to an assay with 5% DMSO and without inhibitor (taken as 100%). Values presented are means of duplicates.

$IC_{50}$ values are determined with several concentrations of the inhibitor using the Hill equation:

$$\log[v_i/(v_o-v_i)] = -n \log[I] + \log K'$$

where $v_o$ is the rate without inhibitor, $v_i$ the rate in the presence of inhibitor and $n$ the Hill number.

Method B: MurE enzyme assay

The compounds are tested for their ability to inhibit the formation of UDP-MurNAc-L-Ala-γ-D-Glu-L-[$^{14}$C]Lys in mixtures (50 μl) containing 0.1 M Tris-HCl, pH 8.6, 15 mM $MgCl_2$, 5 mM ATP, 100 μM UDP-MurNAc-L-Ala-γ-D-Glu, 200 μM L-[$^{14}$C]Lys (50,000 cpm), inhibitor (100 μM), DMSO (5%, v/v), and enzyme from *S. aureus.* After 30 min at 37°C, the reaction is stopped by addition of acetic acid (10 μl). After lyophilization, L-[$^{14}$C]Lys and of UDP-MurNAc-L-Ala-γ-D-Glu-L-[$^{14}$C]Lys are separated by reverse-phase HPLC on Nucleosil 100C18 5U (150×4.6 mm) in 50 mM ammonium formate, pH 4.7, at 0.6 ml/min, and quantitated by on-line scintillation counting using the Quicksafe Flow 2 scintillating fluid. The residual activity is determined with respect to an assay with 5% DMSO and without inhibitor (taken as 100%). Values presented are means of duplicates.

$IC_{50}$ values were determined with several concentrations of the inhibitor using the Hill equation:

$$\log[v_i/(v_o-v_i)] = -n \log[I] + \log K'$$

where $v_o$ is the rate without inhibitor, $v_i$ the rate in the presence of inhibitor and $n$ the Hill number.

**Table 2: Activities of representative compounds tested in an *E. coli* MurD assay and S. *aureus* MurE assay.**

TABLE 2

| **Compound of Example No.** | ***E. coli* MurD** | | ***S. aureus* MurE** | |
|---|---|---|---|---|
| | RA at 100μM inhibitor conc. | $IC_{50}$ (μM) | RA at 100μM inhibitor conc. | $IC_{50}$ (μM) |
| 1 | 77% | >100 | 82% | >100 |

(continued)

| Compound of Example No. | *E. coli* MurD | | *S. aureus* MurE | |
|---|---|---|---|---|
| | RA at 100μM inhibitor conc. | $IC_{50}$ (μM) | RA at 100μM inhibitor conc. | $IC_{50}$ (μM) |
| 2 | 22% | 111 | 7% | 4 |
| 3 | 7% | 24 | 4% | 5 |
| 4 | 26% | 15 | 4% | 4 |
| 5 | 71% | >100 | 10% | 14 |
| 6 | 99% | >100 | 5% | 37 |
| 7 | 98% | >100 | 19% | 7 |
| 8 | 100% | >100 | 23% | 1.5 |
| 9 | 98% | >100 | 17% | 51 |
| 10 | 97% | >100 | 31% | 13 |
| 11 | 95% | >100 | 25% | 4.5 |
| 12 | 78% | >100 | 59% | >100 |
| 13 | 75% | >100 | 90% | >100 |
| 14 | 72% | >100 | 67% | >100 |

## Claims

**1.** A compound of formula

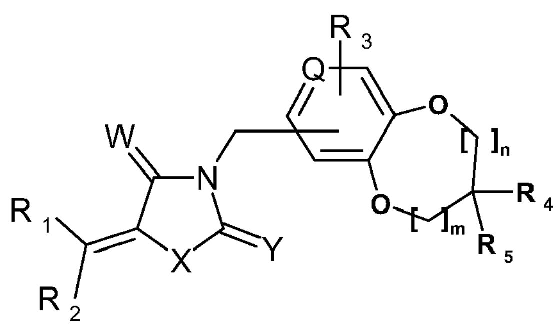

I

wherein

X denotes S, NH, $CH_2$ or O
Y denotes S or O,
W denotes S or O,
Q denotes CH or N,
n is an integer from 0 to 2,
m is an integer from 0 to 2, whereby the sum of n+m is not greater than 2,
R1 and R2 denote independently from each other hydrogen, optionally substituted aryl, optionally substituted heterocyclyl, or an optionally substituted ($C_{1-10}$) aliphatic group,
R3 denotes hydrogen, lower alkyl, halogen, lower alkoxy, nitro, and
R4 and R5 denote independently from each other hydrogen, halogen or alkyl,
with the proviso that if one of R1 and R2 is hydrogen then the respective other one of R1 and R2 does not denote substituted or unsubstituted 2-furyl,

and pharmaceutically acceptable salts of a compound of formula I.

**2.** A compound according to claim 1 wherein $R_4$ is hydrogen and $R_5$ is hydrogen.

**3.** A compound according to any of the preceding claims wherein $R_3$ is hydrogen.

**4.** A compound according to any of the preceding claims wherein Q is CH.

**5.** A compound according to any of the preceding claims wherein one residue of the group consisting of R1 and R2 is hydrogen and the other one is aryl or a monocyclic or bicyclic aromatic heterocyclyl with the proviso that R1 or R2 is not 2-furanyl.

**6.** A compound according to any of the preceding claims wherein n and m are 0.

**7.** A compound according any of the preceding claims as a pharmaceutical.

**8.** The compound according to claim 10 wherein the pharmaceutical is an antibiotic.

**9.** Use of a compound of formula I as defined in claim 1 in the preparation of a medicament for the treatment of bacterial diseases.

**10.** The use according to claim 11 wherein the bacterial disease is caused by a bacterium strain exhibiting MurD and/or MurD ligase activity.

**11.** A process for the preparation of a compound of formula I as defined in claim 1 comprising the steps of

a) reacting a compound of formula

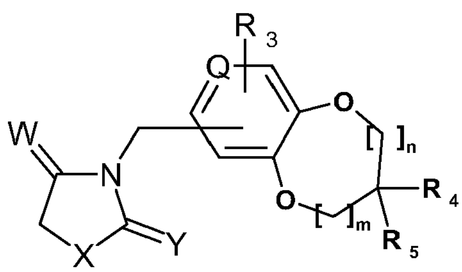

V

wherein W, X, Y, Q, R3, n, m, R4 and R5 are as defined in formula I of claim 1, with an aldehyde or ketone of formula

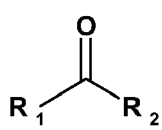

VI

wherein R1 and R2 are defined as in formula I in claim 1
to obtain a compound of formula I, and optionally
c) isolating a compound of formula I.

**12.** A process for the preparation of a compound of formula I as defined in claim 1 comprising the steps of

t) reacting a compound of formula

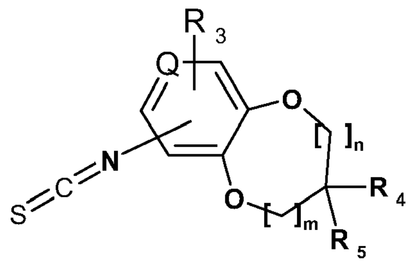

III

wherein Q, R3, R4, R5, n and m are as defined in formula I of claim 1, with a compound of formula

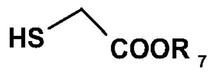

IV

wherein R7 denotes alkyl, to obtain a compound of formula

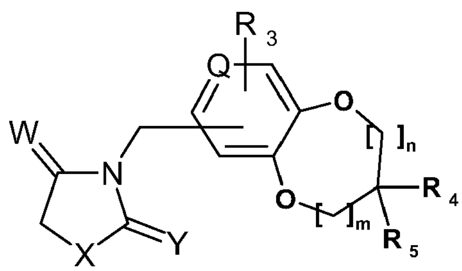

V

wherein W, X, Y, Q, R3, n, m, R4 and R5 are as defined in formula I of claim 1,
b) reacting a compound of formula V as defined in step t) with an aldehyde or ketone of formula

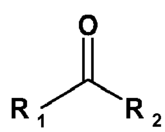

VI

wherein R1 and R2 are defined as in formula I of claim 1, to obtain a compound of formula I, and optionally
c) isolating a compound of formula I.

**13.** A process for the preparation of a compound of formula I as defined in claim 1 wherein X denotes $CH_2$ comprising the steps of

i) reacting a compound of formula

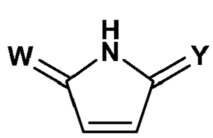

XI

wherein W and Y have the same meaning as in formula I o claim 1with a compound of formula

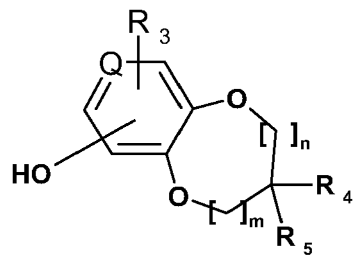

XII

wherein R3, R4, R5, n, m and Q are defined as in formula I of claim 1 to obtain a compound of formula

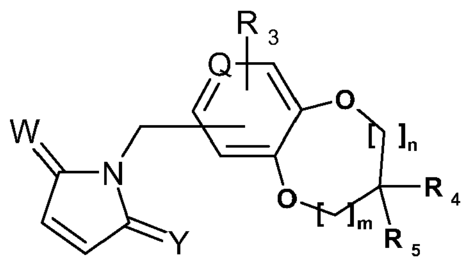

XIII

wherein R3, R4, R5, n, m, Q, W and Y are defined as in formula I of claim 1,
ii) reacting a compound of formula XIII as defined in step i) with a compound of formula

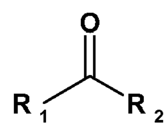

VI

wherein R1 and R2 have the same meaning as defined above in formula I of claim 1 to obtain a compound of formula I wherein X denotes CH, and optionally
iii) isolating a compound of formula I wherein X denotes CH.

**14.** A pharmaceutical composition comprising a compound of formula I as defined in claim 1 or a salt thereof and one or more pharmaceutically acceptable excipient(s).

European Patent Office

# EUROPEAN SEARCH REPORT

Application Number
EP 06 12 2058

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2006/066846 A1 (CELL THERAPEUTICS EUROPE S.R.L., ITALY) 29 June 2006 (2006-06-29) * claims 1,3 * | 1-7 | INV. C07D471/04 C07D417/14 C07D417/06 C07D407/06 A61K31/427 A61P31/04 |
| Y | WO 2004/043955 A (RIGEL PHARMACEUTICALS INC [US]; SINGH RAJINDER [US]; RAMESH USHA V [US) 27 May 2004 (2004-05-27) * page 2, paragraph 7; claim 1; compound 299 * | 1-7 | |
| X | WO 98/53790 A2 (TEXAS BIOTECHNOLOGY CORPORATION, USA) 3 December 1998 (1998-12-03) * compounds 11, 16, 18, 35 * | 1-7 | |
| X | WO 2004/096807 A2 (TIBOTEC PHARMACEUTICALS LTD., IRE.) 11 November 2004 (2004-11-11) example 8, step 2, page 66 | 1-6 | |
| Y | GB 2 387 172 A (PANTHERIX LTD., UK) 8 October 2003 (2003-10-08) * claim 1; table 1 * | 1-14 | TECHNICAL FIELDS SEARCHED (IPC) C07D |
| Y | WO 2005/020990 A1 (CENTRE NATIONAL DE LA RECHERCHE SCIENTIFIQUE CNRS, FR.) 10 March 2005 (2005-03-10) * claims 1,19,26 * | 1-14 | |

-/--

The present search report has been drawn up for all claims

| Place of search | Date of completion of the search | Examiner |
|---|---|---|
| Munich | 14 March 2007 | Schuemacher, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

4

EPO FORM 1503 03.82 (P04C01)

European Patent Office

# EUROPEAN SEARCH REPORT

Application Number
EP 06 12 2058

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HUSAIN M I ET AL: "Synthesis and pharmacological studies of some 3-(N,N-disubstituted aminomethyl(-5-(fluorobenzylidene)-4-oxo-thiazolidin-2-thiones" ACTA PHARMACEUTICA JUGOSLAVICA, SAVEZ FARMACEUTSKIH DRUSTAVA JUGOSLAVIJE, ZAGREB, YU, vol. 36, no. 3, 1986, pages 311-317, XP002086133 ISSN: 0001-6667 scheme 1, table I ----- | 1-14 | |
| A | HU ET AL: "Identification of Selective Inhibitors for the Glycosyltransferase MurG via High-Throughput Screening" CHEMISTRY AND BIOLOGY, CURRENT BIOLOGY, LONDON, GB, vol. 11, no. 5, May 2004 (2004-05), pages 703-711, XP002348827 ISSN: 1074-5521 * figure 3; table 2; compounds 1,2,3 * ----- | 1-14 | TECHNICAL FIELDS SEARCHED (IPC) |

The present search report has been drawn up for all claims

| Place of search | Date of completion of the search | Examiner |
|---|---|---|
| Munich | 14 March 2007 | Schuemacher, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C01)

## ANNEX TO THE EUROPEAN SEARCH REPORT ON EUROPEAN PATENT APPLICATION NO. EP 06 12 2058

This annex lists the patent family members relating to the patent documents cited in the above-mentioned European search report.
The members are as contained in the European Patent Office EDP file on
The European Patent Office is in no way liable for these particulars which are merely given for the purpose of information.

14-03-2007

| Patent document cited in search report | Publication date | Patent family member(s) | Publication date |
|---|---|---|---|
| WO 2006066846 A1 | 29-06-2006 | NONE | |
| WO 2004043955 A | 27-05-2004 | AU 2003291024 A1 | 03-06-2004 |
| | | EP 1597255 A1 | 23-11-2005 |
| WO 9853790 A2 | 03-12-1998 | AU 7684798 A | 30-12-1998 |
| WO 2004096807 A2 | 11-11-2004 | AU 2004234087 A1 | 11-11-2004 |
| | | BR PI0409873 A | 16-05-2006 |
| | | CA 2522990 A1 | 11-11-2004 |
| | | CN 1812992 A | 02-08-2006 |
| | | EP 1625130 A2 | 15-02-2006 |
| | | KR 20060006047 A | 18-01-2006 |
| | | MX PA05011726 A | 23-01-2006 |
| | | US 2006211724 A1 | 21-09-2006 |
| GB 2387172 A | 08-10-2003 | NONE | |
| WO 2005020990 A1 | 10-03-2005 | CN 1607202 A | 20-04-2005 |
| | | EP 1653954 A1 | 10-05-2006 |
| | | FR 2858324 A1 | 04-02-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **A. El ZOEIBY et al.** *Molecular Microbiology,* 2003, vol. 47, 1-12
- **F. M. WALSH et al.** *Current Opinion in Microbiology,* 2004, vol. 7, 439-444
- **ORCHARD M.G et al.** *Bioorg. Med. Chem. Lett.,* 2004, vol. 14, 3975-3978
- **WOOLHOUSE A.D. et al.** *J. Mater. Chem.,* 2001, vol. 11, 2271-2281
- **SIM M.M. et al.** *Bioorg. Med. Chem. Lett.,* 2001, vol. 11, 91-94
- **JAE-KON.** *Bulletin of the Korean Chemical Society,* 2002, vol. 23 (5), 661-662
- **YASUYUKI KITA.** *JOC,* 1998, vol. 63, 6625-6633
- **REDDY TJ et al.** *JOC,* 2002, vol. 67, 4635
- **J. S. BUCK ; W. S. IDE.** *Organic Syntheses, Coll.,* 1943, vol. 2, 622
- *ORGANIC SYNTHESES, COLL.,* 1935, vol. 15, 85
- **M. SCHLOSSER ; J. GORECKA ; E. CASTAGNETTI.** *Eur. J. Org. Chem.,* 2003, 452-462
- **J. A. BERTRAND et al.** *Journal of Molecular Biology,* 1999, vol. 289, 579-590
- **J. VAN HEIJENOORT.** *Natural Product Reports,* 2001, vol. 18, 503-519